# EUROPEAN PATENT SPECIFICATION

(11) **EP 4 562 638 B1**
(45) Date of publication and mention of the grant of the patent: **01.07.2026**
(21) Application number: 23757482.7
(22) Date of filing: 26.07.2023
(51) Int. Cl.: G16B 30/00

(54) **RAPID SINGLE-CELL MULTIOMICS PROCESSING USING AN EXECUTABLE FILE**
SCHNELLE EINZELZELLEN-MULTIOMICS-VERARBEITUNG UNTER VERWENDUNG EINER AUSFÜHRBAREN DATEI
TRAITEMENT MULTIOMIQUE À CELLULE UNIQUE RAPIDE À L'AIDE D'UN FICHIER EXÉCUTABLE

(30) Priority: 26.07.2022 US 202263369482 P
(43) Date of publication of application: 04.06.2025
(73) Proprietor: Illumina, Inc., San Diego, CA 92122 (US)
(72) Inventor: MANDRIC, Igor, San Diego, CA 92122 (US); TRUONG, Sean, San Diego, CA 92122 (US); BRUNDU, Francesco Gavino, San Diego, CA 92122 (US)
(74) Representative: Robinson, David Edward Ashdown
(86) International application number: PCT/US2023/071034
(87) International publication number: WO 2024/026356

(56) References cited:
- WO-A1-2022/051528
- US-A1- 2020 248 255
- US-A1- 2021 332 354
- TOM SMITH ET AL: "UMI-tools: modeling sequencing errors in Unique Molecular Identifiers to improve quantification accuracy", GENOME RESEARCH, vol. 27, no. 3, 18 January 2017 (2017-01-18), US, pages 491 - 499, XP055517852, ISSN: 1088-9051, DOI: 10.1101/gr.209601.116

## Description

### CROSS-REFERENCE TO RELATED APPLICATIONS

The present application claims the benefit of, and priority to, U.S. Provisional Application No. 63/369,482, entitled "RAPID SINGLE-CELL MULTIOMICS PROCESSING USING AN EXECUTABLE FILE," filed on July 26, 2022.

### BACKGROUND

In recent years, biotechnology firms and research institutions have improved hardware and software for (i) sequencing nucleotides that indicate gene expression, accessible chromatin, and methylation for a sample's individual cells and (ii) determining metrics measuring cell-specific gene expression, accessible chromatin, etc. For instance, some existing sequencing machines and sequencing-data-analysis software (together "existing sequencing systems") synthesize oligonucleotides that have been extracted from a sample and placed into library fragments to determine nucleobase calls for nucleotide reads. Such reads may include genomic reads corresponding to deoxyribonucleic acid (DNA) from open chromatin, ribonucleic acid (RNA)-based reads corresponding to a transcriptome, or other nucleotide reads. To illustrate one such genomic read, some existing sequencing systems synthesize Assay for Transposase-Accessible Chromatin (ATAC) reads based on genomic DNA from accessible chromatin that a transposase has identified by inserting adapters into open gDNA regions of a cell.

In some cases, assays for ATAC reads and RNA-based reads can be combined into a multiomics assay that generate metrics corresponding to DNA-based and RNA-based reads. Based on cell barcodes embedded in nucleotide reads, for instance, some existing sequencing systems run a multiomics assay to determine counts of RNA-based reads and ATAC reads for genes and selected genomic regions corresponding read-coverage peaks, respectively, for various cells as indicators of cell-specific gene expression and cell-specific accessible chromatin.

Despite these recent advances, existing sequencing systems that use state-of-the-art technology for multiomics assays still consume excessive computer-processing time and memory to determine cell-specific gene-expression and accessible chromatin metrics respectively using RNA-based reads and ATAC reads. For example, some existing sequencing systems run individual scripts for approximately two hours (or more) to execute a multiomics assay with approximately 50 million RNA-based reads and 200 million ATAC reads and generate the relevant cell-specific metrics. To illustrate, some existing sequencing systems run individual Python scripts for a collection of multiomics software that consumes about two hours to process RNA-based reads and ATAC reads associated with cellular barcodes and unique molecular identifiers (UMIs), correct errors in such barcodes and UNils, align ATAC and transcriptomic reads, count such reads per feature, and filter cells based on counts, respectively, among other such tasks. Accordingly, the individual scripts for various pipelines within such a collection of multiomics software unnecessarily prolong computer processing.

In addition to consuming excessing computing-processing time, existing sequencing systems consume unnecessary memory to run such multiomics assays using RNA-based reads and ATAC reads. For instance, as one pipeline runs using RNA-based reads or another pipeline runs using ATAC reads, some existing sequencing systems store counts for ATAC reads or RNA-based reads, respectively, on a hard drive or disc. When a multiomics assays includes approximately 50 million RNA-based reads and 200 million ATAC reads, corresponding read counts per feature per cell consume considerable memory. By storing on and accessing read counts from a hard drive, existing sequencing systems not only consume unnecessary and slow-accessible memory, but slow down either gene-expression assays or ATAC assays, but also combined multiomics assays.

Document WO2022/051528 A1 discloses methods for distinguishing cell populations from non-cell populations based on classification of cell-associated barcodes in data involving several genomic features from single-cell datasets.

These, along with additional problems and issues exist in existing sequencing systems.

### SUMMARY

This disclosure describes one or more embodiments of systems, methods, and non-transitory computer readable storage media that solve one or more of the problems described above or provide other advantages over the art. According to a first aspect, there is provided a computer-implemented method according to claim 1. According to a second aspect, there is provided a system according to claim 14. According to a third aspect, there is provided a non-transitory computer readable medium according to claim 15. In particular, the disclosed systems can use a single executable file to efficiently run a single-cell multiomics analysis that (i) aligns transcriptomic reads and genomic reads with a reference genome and (ii) jointly filters cellular barcode sequences for cells based on feature-specific, single-cell read counts. To run such an assay using a single executable file, the disclosed systems identify transcriptomic reads and genomic reads for a sample, where such reads comprise different sets of cellular barcode sequences. In some cases, the disclosed systems further use separate invocations of a configurable processor to align the transcriptomic reads and genomics reads with a reference genome. Based on single-cell counts of aligned transcriptomic reads and single-cell counts of aligned genomic reads for target nucleotide sequences within cells of the sample, the disclosed systems select a subset of candidate cells corresponding to a subset of cellular barcode sequences. The disclosed systems further generate, for the sample, single-cell multiomics outputs for individual cells of the selected subset of candidate cells based on the counts of aligned reads.

Additional features and advantages of one or more embodiments of the present disclosure will be set forth in the description which follows, and in part will be obvious from the description, or may be learned by the practice of such example embodiments.

### BRIEF DESCRIPTION OF THE DRAWINGS

The detailed description refers to the drawings briefly described below.
FIG. 1 illustrates a schematic diagram of a computing system in which a multiomics sequencing system can operate in accordance with one or more embodiments of the present disclosure.
FIG. 2 illustrates an existing sequencing system running a gene-expression pipeline and an Assay for Transposase-Accessible Chromatin (ATAC) pipeline as part of a multiomics assay.
FIG. 3 illustrates the multiomics sequencing system performing a single-cell multiomics analysis by (i) aligning transcriptomic reads and genomic reads with a reference genome and (ii) selecting a subset of candidate cells corresponding to a subset of cellular barcode sequences based on feature-specific, single-cell read counts in accordance with one or more embodiments of the present disclosure.
FIG. 4A illustrates the multiomics sequencing system performing a single-cell multiomics analysis by using separate cell filtering for transcriptomic and genomic reads of a sample in accordance with one or more embodiments of the present disclosure.
FIG. 4B illustrates the multiomics sequencing system performing a single-cell multiomics analysis by (i) using different configurable-processor invocations to align transcriptomic reads and genomic reads and (ii) jointly filtering cells based on single-cell counts of both transcriptomic reads and genomic reads in accordance with one or more embodiments of the present disclosure.
FIG. 5 illustrates an overview of the multiomics sequencing system jointly filtering candidate cells by determining thresholds for single-cell UMI-sequence counts and single-cell read counts and clustering candidate cells according to UMI-sequence counts and genomic-read counts in accordance with one or more embodiments of the present disclosure.
FIGS. 6A-6B illustrate knee-plot graphs depicting the multiomics sequencing system determining one or more of a UMI-sequence-count threshold or a genomic-read-count threshold in accordance with one or more embodiments of the present disclosure.
FIG. 7 illustrates the multiomics sequencing system determining UMI-sequence counts and genomic-read counts for candidate cells and deduplicating certain candidate cells in accordance with one or more embodiments of the present disclosure.
FIGS. 8A-8B illustrate graphs depicting the multiomics sequencing system clustering candidate cells based on summed single-cell counts of UMI sequences and genomic reads in accordance with one or more embodiments of the present disclosure.
FIG. 9 illustrates a series of acts for performing a single-cell multiomics analysis in accordance with one or more embodiments of the present disclosure.
FIG. 10 illustrates a block diagram of an example computing device in accordance with one or more embodiments of the present disclosure.

### DETAILED DESCRIPTION

This disclosure describes one or more embodiments of a multiomics sequencing system that can use a single executable file to efficiently run a single-cell multiomics analysis that (i) aligns transcriptomic reads and genomic reads with a reference genome and (ii) jointly filters cellular barcode sequences for cells based on feature-specific, single-cell read counts. To run such a single-cell multiomics assay using a single executable file, the multiomics sequencing system identifies transcriptomic reads and genomic reads for a sample, where such reads comprise different sets of cellular barcode sequences representing candidate cells of the sample. In some cases, the multiomics sequencing system further uses separate configurations of a Field Programmable Gate Array (FPGA) or other configurable processor to align the transcriptomic reads and genomics reads with a reference genome. Based on single-cell counts of aligned transcriptomic reads for genes and single-cell counts of aligned genomic reads for accessible genomic regions corresponding to read-coverage peaks, the multiomics sequencing system selects a subset of candidate cells corresponding to a subset of cellular barcode sequences. The multiomics sequencing system further generates, for the sample, single-cell multiomics outputs for individual cells of the selected subset of candidate cells based on the counts of aligned transcriptomic reads and aligned genomic reads.

As just noted, in some embodiments, the multiomics sequencing system identifies transcriptomic reads and genomic reads that each include different sets of cellular barcode sequences. While the represented cells of the sample are the same or substantially overlap, in some embodiments, the set of cellular barcode sequences incorporated into the transcriptomic reads for a gene-expression assay differ from the set of cellular barcode sequences incorporated into the genomic reads for an ATAC assay or methylation assay. But such different sets can nevertheless be mapped to the same overlapping cells from the sample. Similarly, the multiomics sequencing system can identify transcriptomic and genomic reads generated by a same or different sequencing run or same or different sequencing machine.

Having identified or received such reads, in some embodiments, the multiomics sequencing system invokes a single FPGA or other configurable processor to perform separate alignment of transcriptomic reads and genomic reads with a reference genome. In a first invocation, for instance, the multiomics sequencing system configures a configurable processor to execute a first alignment model (e.g., DNA aligner model) that aligns the genomic reads with the reference genome. In a second invocation, the multiomics sequencing system configures the same configurable processor to execute a second alignment model (e.g., RNA aligner model) that aligns the transcriptomic reads with the reference genome. The type of reads aligned in each configurable-processor invocation can be programmed for any order.

After aligning transcriptomic and genomic reads, as suggested above, the multiomics sequencing system can jointly filter cells of the sample represented by cellular barcode sequences. For instance, the multiomics sequencing system can determine single-cell counts of aligned transcriptomic reads for each gene and single-cell counts of genomic reads for each accessible genomic region corresponding to a read-coverage peak. In some cases, the single-cell counts of aligned transcriptomic reads comprise single-cell counts of unique molecular identifier (UMI) sequences within (or corresponding to) aligned transcriptomic reads. The multiomics sequencing system can further determine summed single-cell counts for aligned transcriptomic reads and summed single-cell counts of aligned genomic reads for each candidate cell. Based on such summed single-cell counts of aligned transcriptomic reads and aligned genomic reads, in some embodiments, the multiomics sequencing system clusters cellular barcode sequences representing candidate cells and selects a subset of candidate cells as more likely representing real biological cells (or valid cells) of the sample.

Having jointly filtered cells, in some embodiments, the multiomics sequencing system generates, for the sample, single-cell multiomics outputs for the selected subset of candidate cells. The single-cell multiomics outputs can include a subset of single-cell counts of aligned transcriptomic reads and a subset of single-cell counts of aligned genomic reads corresponding to the selected subset of candidate cells. As part of an expedited multiomics assay, for instance, the multiomics sequencing system can generate single-cell multiomics outputs for multiple different "omes," such as metrics for a transcriptome indicating gene expression for each candidate cell and metrics for a genome indicating accessible genomic deoxyribonucleic acid (DNA) corresponding to open chromatin.

As indicated above, the multiomics sequencing system provides several technical advantages relative to existing sequencing systems by, for example, improving the computer-processing time and memory consumed to perform a multiomics assays. For example, the multiomics sequencing system expedites the computer-processing time used to perform a multiomics assay using transcriptomic and genomic reads. Unlike existing sequencing systems that use individual scripts to perform separate tasks of a multiomics assay, in some embodiments, the multiomics sequencing system uses a single multiomics executable file to run a single-cell multiomics assay that can (i) align transcriptomic reads and genomic reads with a reference genome and (ii) jointly filter cellular barcode sequences for cells based on feature-specific, single-cell counts for transcriptomic and genomic reads. In some such cases, the multiomics sequencing system runs two separate invocations or configurations of a same configurable processor to align the transcriptomic reads and genomics reads. In part due to running a single multiomics executable file and jointly filtering cells to select a subset of candidate cells, the disclosed multiomics sequencing system reduces the computer-processing time to execute a multiomics assay processing approximately 50 million RNA-based reads and 200 million ATAC reads from over 2 hours by the state-of-the-art systems to approximately 12 minutes.

In addition to or as part of expedited computer processing, in some embodiments, the multiomics sequencing system also improves the memory usage and memory accessibility for performing a multiomics assay using transcriptomic and genomic reads. Unlike existing sequencing systems that run separate scripts and further store and access reads counts on and from a hard drive or disc, in some embodiments, the multiomics sequencing system uses a single multiomics executable file that facilitates storing counts for transcriptomic reads and genomic reads and other data on high-speed storage media, such as random-access memory (RAM). Because some multiomics assays often includes approximately 50 million RNA-based reads and 200 million DNA-based reads, the multiomics sequencing system significantly expedites speed and memory accessibility by running a multiomics executable file that can store and access corresponding read counts per feature per candidate cell from RAM or other high-speed storage media.

As illustrated by the foregoing discussion, the present disclosure utilizes a variety of terms to describe features and advantages of the multiomics sequencing system. As used herein, for example, the term "nucleotide read" (or simply "read") refers to an inferred sequence of one or more nucleobases (or nucleobase pairs) from all or part of a sample nucleotide sequence. Such a sample nucleotide sequence may take the form of a sample genomic sequence from genomic DNA (gDNA), a transcriptomic sequence from complementary DNA (cDNA), a transcriptomic sequence from RNA, or other nucleotide sequence. In particular, a nucleotide read includes a determined or predicted sequence of nucleobase calls for a nucleotide sequence (or group of monoclonal nucleotide sequences) from a sample library fragment corresponding to a sample. For example, in some cases, a sequencing device determines a nucleotide read by generating nucleobase calls for nucleobases passed through a nanopore of a nucleotide-sample slide, determined via fluorescent tagging, or determined from a cluster in a flow cell. A nucleotide read may comprise one or more of a read primer sequence, an indexing sequence, a binding adapter sequence, or a cellular barcode sequence.

Relatedly, as used herein, the term "genomic read" refers to a nucleotide read representing an inferred sequence of nucleobases (or nucleobase pairs) derived from genomic DNA (gDNA) extracted from a sample. For example, a genomic read includes a read comprising gDNA that is (i) extracted from or derived from gDNA extracted from a sample and (ii) part of a sample library fragment corresponding to the sample. In some cases, a genomic read includes reads comprising adapter sequences for Assay for Transposase-Accessible Chromatin (ATAC) reads, which are also called ATAC reads. In some embodiments, genomic reads may include, but are not limited to, DNase 1 hypersensitive sites (DNase) sequencing reads, Formaldehyde-Assisted Isolation of Regulatory Elements (FAIRE) sequencing reads, or Tet-Assisted Bisulfite (TAB) sequencing reads.

Conversely, as used herein, the term "transcriptomic read" refers to a nucleotide read representing an inferred sequence of nucleobases (or nucleobase pairs) that either complement or represent RNA extracted from a sample. For example, a transcriptomic read includes a read comprising cDNA that is (i) synthesized from single-stranded messenger RNA (mRNA) or microRNA (miRNA) or derived from RNA extracted from a sample and (ii) part of a sample library fragment corresponding to the sample. As a further example, a transcriptomic read includes a read comprising RNA (e.g., mRNA, miRNA, transfer RNA (tRNA)) that is (i) extracted from or derived from RNA extracted from a sample and (ii) part of a sample library fragment corresponding to the sample.

As further used herein, the term "cellular barcode sequence" refers to a unique and artificial nucleotide sequence that identifies (or corresponds to) a cell of a sample. In some cases, a cellular barcode sequence includes a unique nucleotide sequence that represents a cell of a sample and that is ligated to a sample's nucleotide sequence (e.g., a gDNA fragment or cDNA fragment) or to another sequence within a sample library fragment. Accordingly, a cellular barcode sequence can be part of a sample library fragment. Similarly, a cellular barcode sequence can be used to sort reads by cell or into different files, among other things.

To illustrate but a few examples, a cellular barcode sequence for an ATAC read may be sixteen nucleobases in length and be represented in single-letter codes for nucleobases as AAACAGCCAAACAACA, AAACAGCCAAACATAG, AAACAGCCAAACCCTA, AAACAGCCAAACCTAT, etc. Further, a cellular barcode sequence for a transcriptomic read may be sixteen nucleobases in length and be represented in single-letter codes for nucleobases as ACAGCGGGTGTGTTAC, ACAGCGGGTTGTTCTT, ACAGCGGGTAACAGGC, ACAGCGGGTGCGCGAA, etc.

As further used herein, the term "multiomics executable file" refers to a file comprising instructions in a programming language that a computing device can directly execute to analyze reads for a multiomics assay. In some cases, a multiomics executable file comprises machine instructions that have been translated from source code by a compiler and that can be executed by a native computing device to analyze reads for a multiomics assay. To illustrate, in some embodiments, a multiomics executable file comprises a compiled binary file deployed on a computing device, such as a remote server or a local server. In some such cases, a multiomics executable file constitutes or is part of a DRAGEN executable software program by Illumina, Inc. As suggested above, a multiomics executable file is not a script or a collection of individual scripts. A script is typically written in a different language (e.g., Python, VBA, Perl) than an executable file and is interpreted from source code or bytecode.

A suggested above, the term "target nucleotide sequence" refers to a nucleotide sequence that is from a sample and that is targeted for detection, measurement, sequencing, or quantification. In particular, a target nucleotide sequence includes a nucleotide sequence from a sample's genome or transcriptome for which an assay is designed to detect, measure, sequence, or quantify. For example, a target nucleotide sequence can include a gene targeted by a gene-expression assay or an accessible genomic region corresponding to a read-coverage peak by an ATAC or by another accessible-chromatin assay.

As indicated above, in some embodiments, the multiomics sequencing system determines a count of genomic reads for an accessible genomic region corresponding to a read-coverage peak. As used herein, the term "read-coverage peak" refers to a set of aligned nucleotide reads that resemble a crest or a pile-up of reads covering or overlapping with a genomic region of a reference genome. In particular, a read-coverage peak refers to a set of genomic reads forming a pile-up of reads aligned with or mapped to an accessible genomic region identified by ATAC as part of a peak-calling process.

Relatedly, the term "accessible genomic region" refers to a genomic region of DNA that is part of accessible chromatin. In particular, an accessible genomic region includes a genomic region from accessible chromatin that a transposase has identified by inserting adapters into open gDNA regions. Accordingly, in some cases, an accessible genomic region comprising a genomic region that corresponds to a read-coverage peak and that is identified by ATAC as part of a peak-calling process.

As further used herein, the term "single-cell multiomics outputs" refers to files, matrices, metrics, or other generated outputs indicating counts or measurements of nucleotide reads (or other biological samples or markers) for a single cell of a sample. For example, a single-cell multiomics output may include counts of aligned transcriptomic reads and/or counts of genomic reads for specific target nucleotide sequences (e.g., genes, accessible genomic regions corresponding to read-cover peaks) in a given cell. As a further example, single-cell multiomics outputs may include metrics for median number of accessible genomic regions corresponding to read-cover peaks (sometimes called median "peaks") per candidate cell, which can be reported in a metrics.csv file. As yet a further example, single-cell multiomics outputs may include a joint cell-by-feature matrix comprising both single-cell counts of aligned transcriptomic reads and single-cell counts of aligned genomic reads for target nucleotide sequences organized by each candidate cell.

As also used herein, the term "reference genome" refers to a digital nucleic acid sequence assembled as a representative example (or representative examples) of genes and other genetic sequences of an organism. Regardless of the sequence length, in some cases, a reference genome represents an example set of genes or a set of nucleic acid sequences in a digital nucleic acid sequence determined as representative of an organism. For example, a linear human reference genome may be GRCh38 (or other versions of reference genomes) from the Genome Reference Consortium. GRCh38 may include alternate contiguous sequences representing alternate haplotypes, such as SNPs and small indels (e.g., 10 or fewer base pairs, 50 or fewer base pairs).

Additionally, as used herein, the term "genomic coordinate" refers to a particular location or position of a nucleotide base within a genome (e.g., an organism's genome or a reference genome). In some cases, a genomic coordinate includes an identifier for a particular chromosome of a genome and an identifier for a position of a nucleotide base within the particular chromosome. For instance, a genomic coordinate or coordinates may include a number, name, or other identifier for a chromosome (e.g., chr1 or chrX) and a particular position or positions, such as numbered positions following the identifier for a chromosome (e.g., chr1:1234570 or chr1:1234570-1234870). Further, in certain implementations, a genomic coordinate refers to a source of a reference genome (e.g., mt for a mitochondrial DNA reference genome or SARS-CoV-2 for a reference genome for the SARS-CoV-2 virus) and a position of a nucleotide-base within the source for the reference genome (e.g., mt:16568 or SARS-CoV-2:29001). By contrast, in certain cases, a genomic coordinate refers to a position of a nucleotide-base within a reference genome without reference to a chromosome or source (e.g., 29727).

As used herein, a "genomic region" refers to a range of genomic coordinates. Like genomic coordinates, in certain implementations, a genomic region may be identified by an identifier for a chromosome and a particular position or positions, such as numbered positions following the identifier for a chromosome (e.g., chr1:1234570-1234870). In various implementations, a genomic coordinate includes a position within a reference genome. In some cases, a genomic coordinate is specific to a particular reference genome.

As used herein, for example, the term "configurable processor" refers to a circuit or chip that can be configured or customized to perform a specific application. For instance, a configurable processor includes an integrated circuit chip that is designed to be configured or customized on site by an end user's computing device to perform a specific application. Configurable processors include, but are not limited to, an application-specific integrated circuit (ASIC), an application-specific standard product (ASSP), a coarse-grained reconfigurable array (CGRA), or an FPGA. By contrast, configurable processors do not include a CPU or GPU. In some embodiments, the multiomics sequencing system uses a configurable processor (e.g., FPGA) or a processor (e.g., CPU) to perform the various embodiments described herein.

Also, as used herein, the term "sample" refers to a target genome or transcriptome (or portion of a genome) from an organism undergoing sequencing. For example, a sample includes a sequence of nucleotides isolated or extracted from a sample organism (or a copy of such an isolated or extracted sequence). In particular, a sample includes a full genome that is isolated or extracted (in whole or in part) from a sample organism and composed of nitrogenous heterocyclic bases. A sample can include a segment of deoxyribonucleic acid (DNA), ribonucleic acid (RNA), or other polymeric forms of nucleic acids or chimeric or hybrid forms of nucleic acids noted below. In some cases, the sample is found in a sample prepared or isolated by a kit and received by a sequencing device. In some cases, the sample is a genomic sample and/or a transcriptomic sample corresponding to a same sample organism.

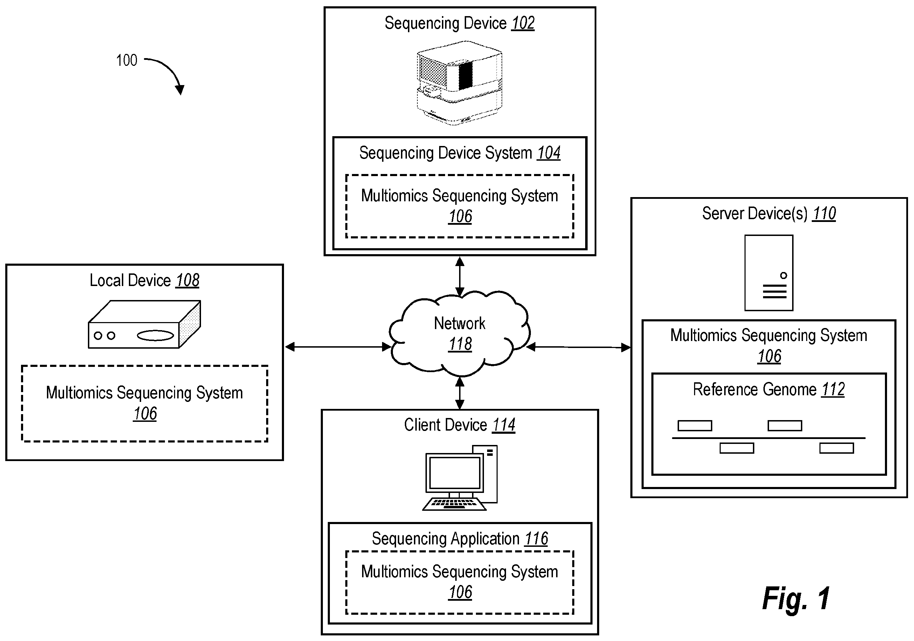

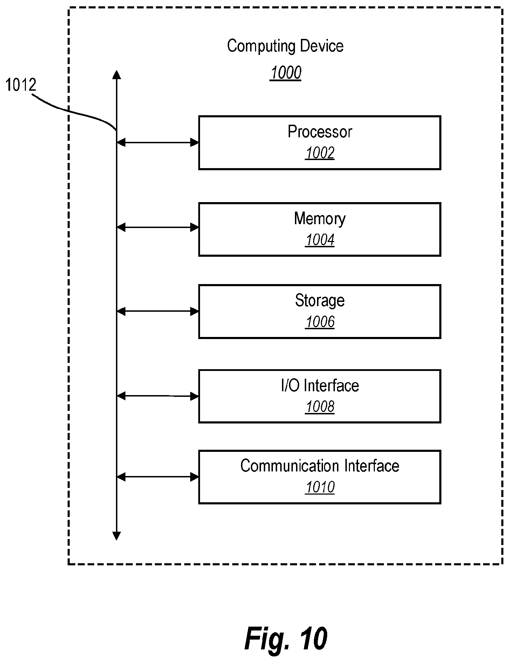

The following paragraphs describe the multiomics sequencing system with respect to illustrative figures that portray example embodiments and implementations. For example, FIG. 1 illustrates a schematic diagram of a computing system 100 in which a multiomics sequencing system 106 operates in accordance with one or more embodiments. As illustrated, the computing system 100 includes a sequencing device 102 connected to a local device 108 (e.g., a local server device), one or more server device(s) 110, and a client device 114. As shown in FIG. 1, the sequencing device 102, the local device 108, the server device(s) 110, and the client device 114 can communicate with each other via a network 118. The network 118 comprises any suitable network over which computing devices can communicate. Example networks are discussed in additional detail below with respect to FIG. 10. While FIG. 1 shows an embodiment of the multiomics sequencing system 106, this disclosure describes alternative embodiments and configurations below.

As indicated by FIG. 1, the sequencing device 102 comprises a computing device and a sequencing device system 104 for sequencing a sample or other nucleic-acid polymer. In some embodiments, by executing the sequencing device system 104 using a processor, the sequencing device 102 analyzes nucleotide fragments or oligonucleotides extracted from samples to generate nucleotide reads or other data utilizing computer implemented methods and systems either directly or indirectly on the sequencing device 102. Such nucleotide reads may include genomic reads, transcriptomic reads, or other reads for a multiomics assay. More particularly, the sequencing device 102 receives nucleotide-sample slides (e.g., flow cells) comprising nucleotide fragments extracted from samples and further copies and determines the nucleobase sequence of such extracted nucleotide fragments to generate nucleotide reads.

In one or more embodiments, the sequencing device 102 utilizes SBS to sequence nucleotide fragments into nucleotide reads and determine nucleobase calls for the nucleotide reads. In addition or in the alternative to communicating across the network 118, in some embodiments, the sequencing device 102 bypasses the network 118 and communicates directly with the local device 108 or the client device 114. By executing the sequencing device system 104, the sequencing device 102 can further store the nucleobase calls as part of base-call data that is formatted as a binary base call (BCL) file and send the BCL file to the local device 108 and/or the server device(s) 110.

As further indicated by FIG. 1, the local device 108 is located at or near a same physical location of the sequencing device 102. Indeed, in some embodiments, the local device 108 and the sequencing device 102 are integrated into a same computing device. The local device 108 may run the multiomics sequencing system 106 to generate, receive, analyze, store, and transmit digital data, such as by receiving base-call data or determining variant calls based on analyzing such base-call data. As shown in FIG. 1, the sequencing device 102 may send (and the local device 108 may receive) base-call data generated during a sequencing run of the sequencing device 102. By executing software in the form of the multiomics sequencing system 106, the local device 108 may align nucleotide reads with a reference genome 112 and determine genetic variants based on the aligned nucleotide reads. The local device 108 may also communicate with the client device 114. In particular, the local device 108 can send data to the client device 114, including a variant call file (VCF) or other information indicating nucleobase calls, sequencing metrics, error data, or other metrics.

As further indicated by FIG. 1, the server device(s) 110 are located remotely from the local device 108 and the sequencing device 102. Similar to the local device 108, in some embodiments, the server device(s) 110 include a version of the multiomics sequencing system 106. Accordingly, the server device(s) 110 may generate, receive, analyze, store, and transmit digital data, such as by receiving base-call data or determining variant calls or single-cell multiomics outputs based on analyzing such base-call data. As indicated above, the sequencing device 102 may send (and the server device(s) 110 may receive) base-call data from the sequencing device 102. The server device(s) 110 may also communicate with the client device 114. In particular, the server device(s) 110 can send data to the client device 114, including VCFs, single-cell multiomics outputs, or other sequencing related information.

In some embodiments, the server device(s) 110 comprise a distributed collection of servers, where the server device(s) 110 include a number of server devices distributed across the network 118 and located in the same or different physical locations. Further, the server device(s) 110 can comprise a content server, an application server, a communication server, a web-hosting server, or another type of server.

As indicated above, as part of the server device(s) 110 or the local device 108, the multiomics sequencing system 106 can run a single-cell multiomics assay that (i) aligns transcriptomic reads and genomic reads with a reference genome and (ii) jointly filters cellular barcode sequences for cells based on feature-specific, single-cell read counts. For instance, the multiomics sequencing system 106 can identify transcriptomic reads and genomic reads corresponding to a sample, where the transcriptomic reads comprise a first set of cellular barcode sequences and the genomic reads comprise a second set of cellular barcode sequences. The multiomics sequencing system 106 further configures a configurable processor to (i) align the transcriptomic reads with a reference genome as part of a first configurable-processor invocation and (ii) align genomics reads with the reference genome as part of a second configurable-processor invocation. Based on single-cell counts of aligned transcriptomic reads and single-cell counts of aligned genomic reads for target nucleotide sequences within cells of the sample, the multiomics sequencing system 106 jointly filters cells by selecting a subset of candidate cells corresponding to a subset of cellular barcode sequences. Based on the counts of aligned transcriptomic reads and aligned genomic reads, the multiomics sequencing system 106 generates single-cell multiomics outputs for individual cells of the selected subset of candidate cells.

As further illustrated and indicated in FIG. 1, by executing a sequencing application 116, the client device 114 can generate, store, receive, and send digital data. In particular, the client device 114 can receive sequencing data from the local device 108 or receive call files (e.g., BCL) and sequencing metrics from the sequencing device 102. Furthermore, the client device 114 may communicate with the local device 108 or the server device(s) 110 to receive a VCF or files for a joint cell-by-feature matrix, genotype calls, and/or other metrics, such as a base-call-quality metrics or pass-filter metrics. The client device 114 can accordingly present or display information pertaining to single-cell multiomics outputs, read counts, genotype calls, or variant calls within a graphical user interface of the sequencing application 116 to a user associated with the client device 114. For example, the client device 114 can present single-cell multiomics outputs from a joint cell-by-feature matrix within a graphical user interface of the sequencing application 116.

Although FIG. 1 depicts the client device 114 as a desktop or laptop computer, the client device 114 may comprise various types of client devices. For example, in some embodiments, the client device 114 includes non-mobile devices, such as desktop computers or servers, or other types of client devices. In yet other embodiments, the client device 114 includes mobile devices, such as laptops, tablets, mobile telephones, or smartphones. Additional details regarding the client device 114 are discussed below with respect to FIG. 10.

As further illustrated in FIG. 1, the client device 114 includes the sequencing application 116. The sequencing application 116 may be a web application or a native application stored and executed on the client device 114 (e.g., a mobile application, desktop application). The sequencing application 116 can include instructions that (when executed) cause the client device 114 to receive data from the multiomics sequencing system 106 and present, for display at the client device 114, base-call data or data from a VCF or single-cell-metrics file.

As further illustrated in FIG. 1, a version of the multiomics sequencing system 106 may be located and implemented (e.g., entirely or in part) on the client device 114 or the sequencing device 102. In yet other embodiments, the multiomics sequencing system 106 is implemented by one or more other components of the computing system 100, such as the local device 108. In particular, the multiomics sequencing system 106 can be implemented in a variety of different ways across the sequencing device 102, the local device 108, the server device(s) 110, and the client device 114. For example, the multiomics sequencing system 106 can be downloaded from the server device(s) 110 to the multiomics sequencing system 106 and/or the local device 108 where all or part of the functionality of the multiomics sequencing system 106 is performed at each respective device within the computing system 100.

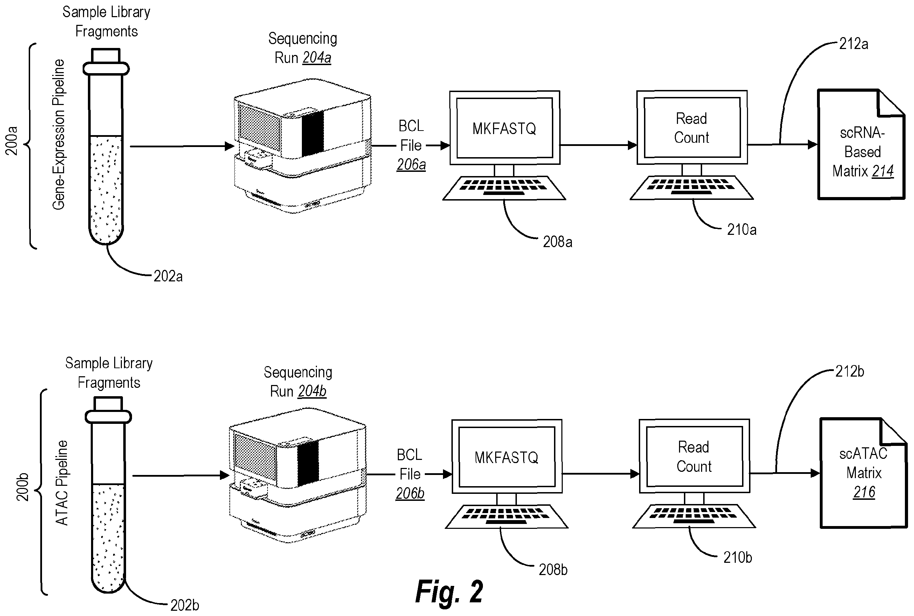

As indicated above, some existing sequencing systems perform multiomics assays. As shown in FIG. 2, for instance, an existing sequencing system runs a gene-expression pipeline 200a and an ATAC pipeline 200b as part of a multiomics assay. By running the gene-expression pipeline 200a and the ATAC pipeline 200b, the existing sequencing system determines single-cell RNA-based matrix 214 comprising counts of RNA-based reads per feature in individual cells and single-cell ATAC matrix 216 comprising counts of ATAC reads per feature in individual cells. Such single-cell RNA-based-read counts and single-cell ATAC-read counts represent indicators of cell-specific gene expression and cell-specific accessible chromatin, respectively. But the gene-expression pipeline 200a and the ATAC pipeline 200b run separate scripts that cause the existing sequencing system to consume excessive computer-processing time and memory to perform the multiomics assay.

As part of the gene-expression pipeline 200a, for instance, the existing sequencing system determines RNA-based reads from sample library fragments 202a and generates an RNA-based-read sequencing file comprising read data. In particular, the existing sequencing system includes a sequencing device that receives a nucleotide-sample slide (e.g.., flow cell) comprising the sample library fragments 202a corresponding to RNA extracted from one or more samples used to detect gene expression. The existing sequencing system further performs a sequencing run 204a to determine nucleobase calls from the sample library fragments 202a and generate RNA-based reads. After the sequencing run 204a, the existing sequencing system sends a BCL file 206a from the sequencing device to a computing device for processing. In particular, the computing device runs a demultiplexing script 208a to demultiplex the BCL file 206a into a sequencing file, such as a FAST-ALL Q (FASTQ) file.

As further part of the gene-expression pipeline 200a in FIG. 2, the existing sequencing system determines single-cell read counts of RNA-based reads for genes of individual cells. For instance, the existing sequencing system corrects errors in cellular barcode sequences and UMI sequences from the RNA-based reads, aligns RNA-based reads with a reference genome, and runs a read-counting script 210a to determine RNA-based-read counts for particular genes within individual cells. Based on the single-cell read counts of RNA-based reads, the existing sequencing system runs an analysis script 212a to filter cellular barcode sequences representing probable cells of a sample and to generate the single-cell RNA-based matrix 214 comprising counts of RNA-based reads per gene in individual cells.

By contrast, as part of the ATAC pipeline 200b, the existing sequencing system determines ATAC reads from sample library fragments 202b and generates an ATAC-read sequencing file comprising read data. In particular, the sequencing device receives a nucleotide-sample slide comprising the sample library fragments 202b corresponding to accessible-chromatin DNA used to assess genome-wide chromatin accessibility in individual cells. The existing sequencing system further performs a sequencing run 204b to determine nucleobase calls from the sample library fragments 202b to generate ATAC reads. After the sequencing run 204b, the existing sequencing system sends a BCL file 206b from the sequencing device to a computing device for processing. In particular, the computing device again runs a demultiplexing script 208b to demultiplex the BCL file 206b into a sequencing file, such as a FASTQ file.

As further part of the ATAC pipeline 200b in FIG. 2, the existing sequencing system determines single-cell read counts of ATAC reads for genomic regions corresponding to read-coverage peaks for DNA within individual cells. For instance, the existing sequencing system corrects errors in cellular barcode sequences from ATAC reads, aligns ATAC reads with a reference genome, and runs a read-counting script 210b to determine read counts for particular genomic regions corresponding to read-coverage peaks for DNA within individual cells. Based on the single-cell read counts of ATAC reads, the existing sequencing system runs an analysis script 212b to filter cellular barcode sequences representing probable cells of a sample and to generate the single-cell ATAC matrix 216 comprising counts of ATAC reads per genomic region corresponding to a read-coverage peak.

By running separate demultiplexing scripts, read-counting scripts, analysis scripts, or other scripts, the existing sequencing system consumes approximately two hours (or more) of computer-processing time to execute a multiomics assay with approximately 50 million RNA-based reads and 200 million ATAC reads. The separate scripts accordingly prolong analysis, isolate similar processes, and sometimes force the existing sequencing system to store data, such as read counts, on a hard drive for later and slower access for analysis.

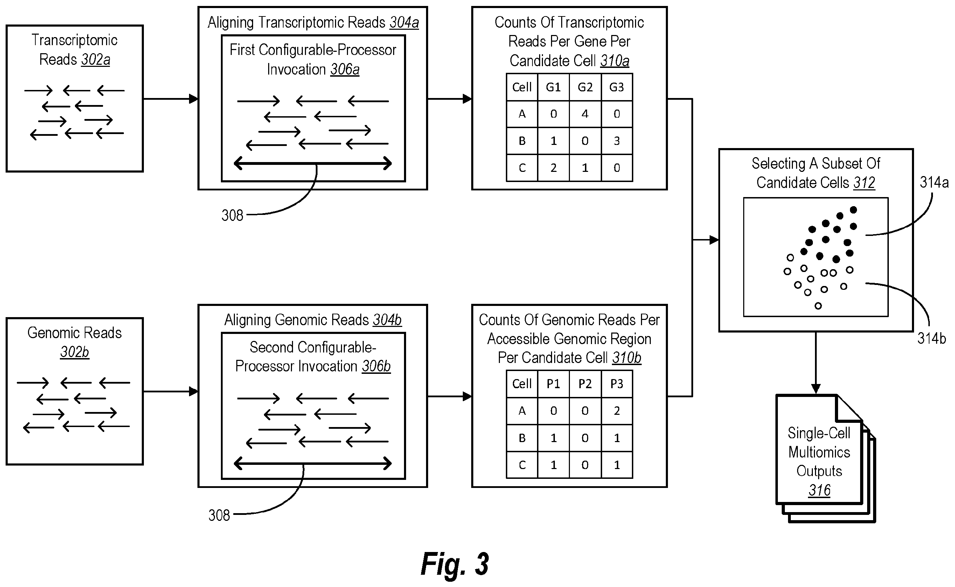

Unlike the separate scripts of existing sequencing systems, in some embodiments, the multiomics sequencing system 106 executes a multiomics executable file to perform a multiomics analysis on reads representing a sample's genome and transcriptome. In accordance with one or more embodiments, FIG. 3 depicts an overview of the multiomics sequencing system 106 performing a single-cell multiomics analysis in part by (i) aligning transcriptomic reads and genomic reads corresponding to a sample with a reference genome using different configurable-processor invocations and (ii) selecting a subset of candidate cells corresponding to a subset of cellular barcode sequences based on feature-specific, single-cell read counts. The following paragraphs describe acts depicted in FIG. 3 performed or facilitated by the multiomics sequencing system 106 executing a single multiomics executable file.

As shown in FIG. 3, for instance, the multiomics sequencing system 106 identifies transcriptomic reads 302a and genomic reads 302b corresponding to a sample. For instance, in some embodiments, the multiomics sequencing system 106 receives or generates a transcriptomic-read sequencing file (e.g., FASTQ) comprising the transcriptomic reads 302a and a genomic-read sequencing file (e.g., FASTQ) comprising the genomic reads 302b. Based on the encoded sequence of corresponding sample library fragments, the transcriptomic reads 302a and the genomic reads 302b each include a cellular barcode sequence representing a cell of a sample. In some embodiments, however, the transcriptomic reads 302a includes a different set of cellular barcode sequences than the genomic reads 302b that the multiomics sequencing system 106 associates with the same corresponding cells.

After identifying the transcriptomic reads 302a and genomic reads 302b, as further shown in FIG. 3, the multiomics sequencing system 106 executes instructions for aligning transcriptomic reads 304a and aligning genomic reads 304b with a reference genome 308. In some embodiments, the multiomics sequencing system 106 performs a first configurable-processor invocation 306a to align the transcriptomic reads 302a with the reference genome 308 and the second configurable-processor invocation 306b to align the genomic reads 302b with the reference genome 308. As explained further below, as part of each of the first configurable-processor invocation 306a and the second configurable-processor invocation 306b, the multiomics sequencing system 106 sends a bit stream representing an alignment model and data representing the reference genome 308 to a configurable processor. While this disclosure describes the first configurable-processor invocation 306a for the transcriptomic reads 302a and the second configurable-processor invocation 306b for the genomic reads 302b, the multiomics sequencing system 106 can execute invocations and align the transcriptomic reads 302a and the genomic reads 302b in any order.

Having aligned the transcriptomic reads 302a and the genomic reads 302b, as further shown in FIG. 3, the multiomics sequencing system 106 determines counts of transcriptomic reads per gene per candidate cell 310a and counts of genomic reads per accessible genomic region per candidate cell 310b. For each set of cellular barcode sequences representing a cell, for instance, the multiomics sequencing system 106 determines single-cell counts of aligned transcriptomic reads for each gene and single-cell counts of aligned genomic reads for each accessible genomic region corresponding to a read-coverage peak. In some embodiments, the multiomics sequencing system 106 (i) determines single-cell counts of aligned transcriptomic reads for each gene by determining single-cell counts of UMI sequences for each gene and (ii) determines single-cell counts of aligned genomic reads for each accessible genomic region by determining single-cell counts of read fragments from genomic reads aligned with each accessible genomic region corresponding to a read-coverage peak.

As depicted in FIG. 3, in some cases, the multiomics sequencing system 106 determines such single-cell counts of aligned transcriptomic reads and single-cell counts of genomic reads using an intermediate matrix. While FIG. 3 depicts an intermediate matrix corresponding to counts of transcriptomic reads and counts of genomic reads, in certain implementations, the multiomics sequencing system 106 uses a consolidated intermediate matrix to determine single-cell counts of both transcriptomic reads and genomic reads per target nucleotide sequence. By using such a consolidated intermediate matrix, the multiomics sequencing system 106 can match or correlate cells represented by different cellular barcode sequences and, in some cases, deduplicate candidate cells exhibiting a same number of single-cell counts of transcriptomic reads and genomic reads.

After determining single-cell counts of transcriptomic reads and genomic reads, in some embodiments, the multiomics sequencing system 106 selects a subset of candidate cells 312 based on the single-cell counts of aligned transcriptomic reads and aligned genomic reads per target nucleotide sequence. For example, in some embodiments, the multiomics sequencing system 106 sums such single-cell counts to determine summed counts of aligned transcriptomic reads for each candidate cell and summed counts of aligned genomic reads for each candidate cell. As noted above, in some cases, the summed single-cell counts of aligned transcriptomic reads comprise summed single-cell counts of UMI sequences within (or corresponding to) aligned transcriptomic reads.

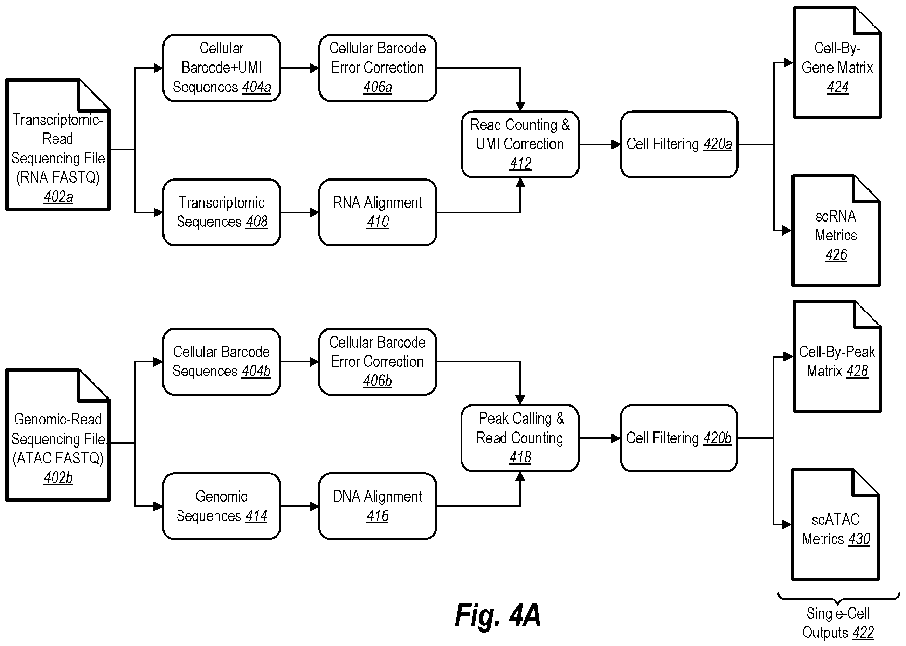

While this disclosure refers to counts of UMI sequences and a UMI-sequence-count threshold below with respect to FIGS. 4A-8B, in some embodiments, the multiomics sequencing system 106 may likewise determine and use counts of aligned transcriptomic reads and a transcriptomic-read-count threshold. Similarly, while this disclosure refers to counts of genomic reads and a genomic-read-count threshold below with respect to FIGS. 4A-8B, in some embodiments, the multiomics sequencing system 106 may likewise determine and use counts of read fragments from transcriptomic reads and a genomic-read-fragment-count threshold.

Based on such summed single-cell counts, the multiomics sequencing system 106 clusters cellular barcode sequences representing candidate cells into a selected cluster of candidate cells 314a and a non-selected cluster of candidate cells 314b. Accordingly, in certain embodiments, the multiomics sequencing system 106 selects a subset of candidate cells as more likely representing real biological cells (or valid cells) of the sample when the selected subset of candidate cells satisfies one or both of a threshold summed count of aligned transcriptomic reads (e.g., UMI sequences) per candidate cell and a threshold summed count of aligned genomic reads relative to a non-selected cluster of candidate cells.

Having selected a subset of candidate cells, as further shown in FIG. 3, the multiomics sequencing system 106 generates, for the sample, single-cell multiomics outputs 316 for the selected subset of candidate cells. The single-cell multiomics outputs 316 can include files reporting a subset of single-cell counts of aligned transcriptomic reads and a subset of single-cell counts of aligned genomic reads corresponding to the selected subset of candidate cells. In some cases, the multiomics sequencing system 106 consolidates single-cell metrics for a transcriptome and genome of the sample into a joint cell-by-feature matrix. For instance, the joint cell-by-feature matrix may comprise both single-cell counts of aligned transcriptomic reads and single-cell counts of aligned genomic reads for target nucleotide sequences organized by each candidate cell within the selected subset of candidate cells.

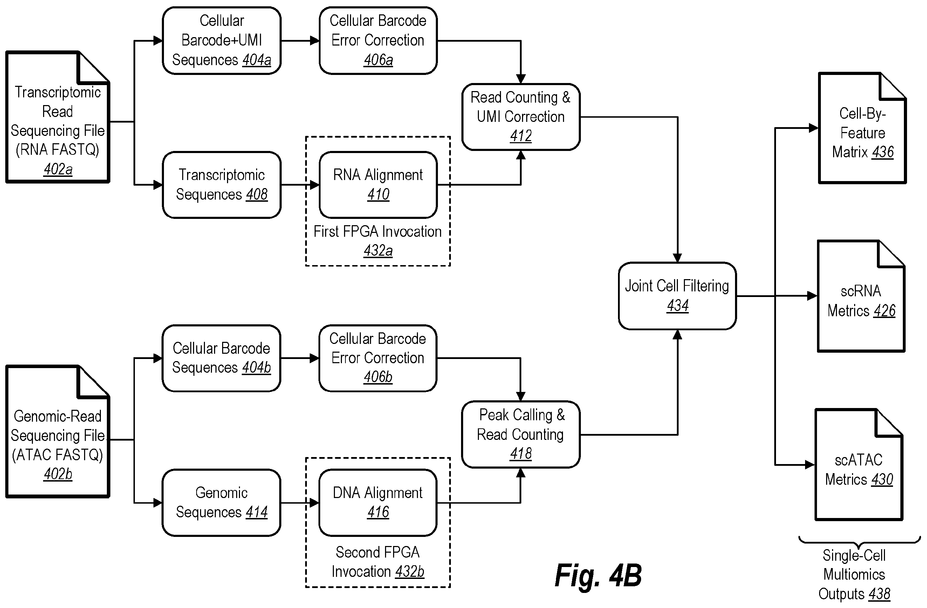

As indicated above, the multiomics sequencing system 106 can integrate analyses for a sample's transcriptome and genome into a single pipeline. In accordance with one or more embodiments, FIG. 4A depicts the multiomics sequencing system 106 performing a single-cell multiomics analysis by using separate cell filtering for transcriptomic and genomic reads of a sample. Further in accordance with one or more embodiments, FIG. 4B depicts the multiomics sequencing system 106 performing a single-cell multiomics analysis by (i) using different configurable-processor invocations to align transcriptomic reads and genomic reads and (ii) jointly filtering cells based on single-cell counts of both transcriptomic reads and genomic reads.

As shown in FIG. 4A, for example, the multiomics sequencing system 106 performs an analysis of transcriptomic reads for a gene-expression assay. In particular, the multiomics sequencing system 106 receives and processes a transcriptomic-read sequencing file 402a, such as a FASTQ file for RNA. The transcriptomic-read sequencing file 402a comprises data for transcriptomic reads, such as single-letter codes representing transcriptomic sequences, cellular barcode sequences, and unique molecular identifier (UMI) sequences, as well as corresponding data fields or headers. After receiving the transcriptomic-read sequencing file 402a, the multiomics sequencing system 106 identifies cellular barcode sequences and UMI sequences 404a as well as transcriptomic sequences 408 within transcriptomic reads. As indicated above, in some embodiments, each sample library fragment for a transcriptomic read includes a cellular barcode sequence representing a candidate cell of a sample, a UMI sequence representing a particular sample library fragment, and a transcriptomic sequence extracted or derived from a transcriptome of a sample's cell, such as a cDNA sequence or an RNA sequence. Based on fields or headers within a FASTQ file or other transcriptomic-read sequencing file, the multiomics sequencing system 106 can identify and differentiate among a cellular barcode sequence, a UMI sequence, and a transcriptomic sequence.

After identifying cellular barcode sequences from transcriptomic reads, as further shown in FIG. 4A, the multiomics sequencing system 106 performs cellular barcode error correction 406a to correct for any sequencing errors of the cellular barcode sequences. For example, in some embodiments, the multiomics sequencing system 106 accesses a whitelist or database of potential cellular barcode sequences for transcriptomic reads. The multiomics sequencing system 106 further compares (i) the identified cellular barcode sequences from sequenced transcriptomic reads with (ii) the whitelist or database of potential cellular barcode sequences to detect differences. If an identified cellular barcode sequence matches a potential cellular barcode sequence from the whitelist, the multiomics sequencing system 106 does not alter the identified cellular barcode sequence. If an identified cellular barcode sequence does not match a potential cellular barcode sequence from the whitelist, however, the multiomics sequencing system 106 alters or corrects the identified cellular barcode sequence to include the same single-letter code as a closest potential cellular barcode sequence from the whitelist within a threshold number of base differences. In some embodiments, the multiomics sequencing system 106 discards transcriptomic reads when its cellular barcode sequence differs from a closest-matching potential cellular barcode sequence beyond the threshold number of base differences.

In addition to correcting cellular barcode sequences of transcriptomic reads, as further shown in FIG. 4A, the multiomics sequencing system 106 performs RNA alignment 410. For example, the multiomics sequencing system 106 uses an alignment model to align transcriptomic sequences from transcriptomic reads with corresponding reference sequences within a reference genome based on alignment score, such as a Smith-Waterman score. In some embodiments, the alignment model includes DRAGEN RNA-Seq spliced aligner by Illumina, Inc. or other RNA alignment model. As suggested above, in some cases, the multiomics sequencing system 106 uses an FPGA or other configurable processor to run the alignment model by (i) mapping seed sequences (e.g., 15-20 nucleobases) from a transcriptomic sequence to candidate reference sequences within a reference genome, (ii) extending the seed sequences to align the transcriptomic sequence with the reference sequences, and (iii) determining which reference sequence exhibits a highest alignment score with the transcriptomic sequence.

After aligning transcriptomic sequences from transcriptomic reads, as further shown in FIG. 4A, the multiomics sequencing system 106 also performs read counting and UMI correction 412. To perform UMI correction, in some embodiments, the multiomics sequencing system 106 determines a count of transcriptomic reads comprising each particular UMI sequence and ranks each UMI sequence according to its corresponding count of transcriptomic reads. The multiomics sequencing system 106 further compares the corresponding count of transcriptomic reads of each UMI sequence to a threshold read count. If the corresponding count of transcriptomic reads for an identified UMI sequence satisfies the threshold read count, the multiomics sequencing system 106 does not alter or correct the identified UMI sequence. If the corresponding count of transcriptomic reads for an identified UMI sequence fails to satisfy the threshold read count, however, the multiomics sequencing system 106 further determines whether the identified UMI sequence satisfies (or is within) a threshold number of base differences of a closest UMI sequence that satisfies the threshold read count, where the closest UMI sequence represents one or more other UMI sequences with a fewest number of base differences from the identified UMI sequence. When the identified UMI sequence satisfies (or is within) the threshold number of base differences of the closest UMI sequence, the multiomics sequencing system 106 alters or corrects the identified UMI sequence to include the same single-letter code as the closest UMI sequence that satisfies the threshold read count. In some embodiments, as above, the multiomics sequencing system 106 discards transcriptomic reads when its identified UMI sequence differs from a closest-matching UMI sequence beyond the threshold number of base differences.

As further suggested above, in some embodiments, the multiomics sequencing system 106 also performs read counting per UMI sequence for each candidate cell represented by a cellular barcode sequence. For each cellular barcode sequence representing a candidate cell, for instance, the multiomics sequencing system 106 determines single-cell counts of aligned transcriptomic sequences from transcriptomic reads for each gene. In some cases, for instance, the multiomics sequencing system 106 determines such single-cell counts of aligned transcriptomic reads using an intermediate matrix comprising columns for genes, rows for cellular barcode sequences, and a number of aligned transcriptomic reads in each matrix cell.

After UMI correction and reading counting, as further shown in FIG. 4A, the multiomics sequencing system 106 performs cell filtering 420a based on single-cell counts of transcriptomic reads per gene. For example, in some embodiments, the multiomics sequencing system 106 clusters cellular barcode sequences representing candidate cells into a selected cluster of candidate cells and a non-selected cluster of candidate cells based on a threshold of UMI-sequence counts per candidate cell for a set of genes. Based on a selected subset of candidate cells satisfying the threshold for UMI-sequence counts per candidate cell, in certain embodiments, the multiomics sequencing system 106 selects the corresponding subset of candidate cells as more likely representing real biological cells (or valid cells) of the sample.

Having filtered cells based on transcriptomic-read counts, in some embodiments, the multiomics sequencing system 106 further generates single-cell metrics 422 for transcriptomic reads. As shown in FIG. 4A, for instance, the multiomics sequencing system 106 generates a cell-by-gene matrix 424 comprising a count of transcriptomic reads corresponding to genes within each cell of the selected subset of candidate cells. Additionally, the multiomics sequencing system 106 generates single-cell RNA metrics 426 comprising statistics corresponding to the gene-expression assay, such as number of transcriptomic reads per candidate cell and number of sequencing runs.

In addition to analyzing transcriptomic reads for the gene-expression assay, as further shown in FIG. 4A, the multiomics sequencing system 106 performs an analysis of genomic reads for an accessible-chromatin assay, such as ATAC. In particular, the multiomics sequencing system 106 receives and processes a genomic-read sequencing file 402b, such as a FASTQ file for DNA. The genomic-read sequencing file 402b comprises data for genomic reads, such as single-letter codes representing genomic sequences and cellular barcode sequences, as well as corresponding data fields or headers. After receiving the genomic-read sequencing file 402b, the multiomics sequencing system 106 identifies cellular barcode sequences 404b and genomic sequences 414 within genomic reads, such as ATAC reads. As indicated above, in some embodiments, each sample library fragment for a genomic read includes a cellular barcode sequence representing a candidate cell of a sample and a genomic sequence extracted or derived from gDNA of a sample's cell, such as a gDNA sequence. Based on fields or headers within a FASTQ file or other genomic-read sequencing file, the multiomics sequencing system 106 can identify and differentiate among a cellular barcode sequence and a transcriptomic sequence (or other sequences) from a sample library fragment.

After identifying cellular barcode sequences from genomic reads, as further shown in FIG. 4A, the multiomics sequencing system 106 performs cellular barcode error correction 406b to correct for any sequencing errors of the cellular barcode sequences. In some embodiments, the multiomics sequencing system 106 follows the same or similar process as the cellular barcode error correction 406a for the cellular barcode error correction 406b. In particular, the multiomics sequencing system 106 compares identified cellular barcode sequences from sequenced genomic reads with a whitelist or database of potential cellular barcode sequences to detect differences. The multiomics sequencing system 106 alters or corrects an identified cellular barcode sequence to include the same single-letter code as a closest potential cellular barcode sequence from the whitelist within a threshold number of base differences-when the identified cellular barcode sequence does not match a potential cellular barcode sequence from the whitelist.

In addition to correcting cellular barcode sequences of genomic reads, as further shown in FIG. 4A, the multiomics sequencing system 106 performs DNA alignment 416. For example, the multiomics sequencing system 106 uses an alignment model to align genomic sequences from genomic reads with corresponding reference sequences within a reference genome based on alignment score. For instance, in some embodiments, the alignment model includes DRAGEN DNA-Seq aligner by Illumina, Inc. or another DNA alignment model. In some cases, the multiomics sequencing system 106 uses an FPGA or other configurable processor to run the alignment model by following the seed-and-extend approach for seed sequences described above, but for aligning genomic sequences with references sequences of the reference genome rather than transcriptomic sequences.

After aligning genomic sequences from genomic reads, as further shown in FIG. 4A, the multiomics sequencing system 106 also performs peak calling and read counting 418. For example, the multiomics sequencing system 106 executes a statistical algorithm to identify accessible genomic regions corresponding to "read-coverage peaks" of aligned genomic reads. In some embodiments, the accessible genomic regions represent the DNA regions of open chromatin identified by ATAC. To identify such accessible genomic regions, in some cases, the multiomics sequencing system 106 uses a peak caller to perform peak calling, such as Genome wide Event finding and Motif discovery (GEM), Model-based Analysis for ChIP-Seq version 2 (MACS2), Bayesian Change Point (BCP), or MUltiScale enrichment Calling for ChIP-Seq (MUSIC).

As suggested above, the multiomics sequencing system 106 also performs read counting per cellular barcode sequence for each candidate cell. For each cellular barcode sequence representing a candidate cell, for instance, the multiomics sequencing system 106 determines single-cell counts of aligned genomic sequences from genomic reads for each accessible genomic region corresponding to a read-coverage peak. In some cases, for instance, the multiomics sequencing system 106 determines such single-cell counts of aligned genomic reads using an intermediate matrix comprising columns for accessible genomic regions or "peaks," rows for cellular barcode sequences, and a number of aligned genomic reads in each matrix cell.

After peak calling and reading counting, as further shown in FIG. 4A, the multiomics sequencing system 106 performs cell filtering 420b based on single-cell counts of genomic reads per accessible genomic region. For example, in some embodiments, the multiomics sequencing system 106 clusters cellular barcode sequences representing candidate cells into a selected cluster of candidate cells and a non-selected cluster of candidate cells based on a threshold of genomic-read counts per candidate cell for a set of accessible genomic regions. Based on a selected subset of candidate cells satisfying the threshold for genomic-read counts per candidate cell, in certain embodiments, the multiomics sequencing system 106 selects the corresponding subset of candidate cells as more likely representing real biological cells (or valid cells) of the sample.

Having filtered cells based on genomic-read counts, in some embodiments, the multiomics sequencing system 106 further generates the single-cell metrics 422 for genomic reads in addition to transcriptomic reads. As shown in FIG. 4A, for instance, the multiomics sequencing system 106 generates a cell-by-peak matrix 428 comprising a count of genomic reads corresponding to accessible genomic regions within each cell of the selected subset of candidate cells. Additionally, in some embodiments, the multiomics sequencing system 106 generates single-cell ATAC metrics 430 comprising statistics corresponding to the accessible-chromatin assay, such as number of genomic reads per candidate cell and number of sequencing runs.

In contrast to the multiomics analysis with separate cell filtering depicted in FIG. 4A, FIG. 4B depicts the multiomics sequencing system 106 performing a single-cell multiomics analysis by (i) using different configurable-processor invocations to align transcriptomic reads and genomic reads with a reference genome and (ii) jointly filtering cells by selecting a subset of candidate cells corresponding to a subset of cellular barcode sequences based on single-cell counts of both transcriptomic reads and genomic reads. To perform the single-cell multiomics analysis in FIG. 4B, in some embodiments, the multiomics sequencing system 106 performs the same actions depicted in FIG. 4A and described above, except for certain modifications described in the following paragraphs. The following paragraphs describe acts depicted in FIG. 4B performed or facilitated by the multiomics sequencing system 106 executing a single multiomics executable file, including the acts in FIG. 4B that are repeated from FIG. 4A.

After receiving and identifying different sequences in one or both of the transcriptomic-read sequencing file 402a and genomic-read sequencing file 402b, as shown in FIG. 4B, the multiomics sequencing system 106 uses a first FPGA invocation 432a to perform the RNA alignment 410 and a second FPGA invocation 432b to perform the DNA alignment 416. During the different FPGA invocations, the multiomics sequencing system 106 sends different bitstreams representing the corresponding alignment model (e.g., RNA alignment model or DNA alignment model) and sends data representing the reference genome twice to an FPGA board or other configurable processor. While this disclosure describes the first FPGA invocation 432a for the RNA alignment 410 and the second FPGA invocation 432b for the DNA alignment 416, the multiomics sequencing system 106 can execute FPGA invocations and align the transcriptomic sequences 408 and the genomic sequences 414 in any order (e.g., aligning genomic sequences with a reference genome in a first FPGA invocation and aligning transcriptomic sequences with the reference genome in a second FPGA invocation).

As part of the first FPGA invocation 432a to perform the RNA alignment 410, for instance, the multiomics sequencing system 106 (i) sends a bit stream encoding for a first alignment model to the FPGA to reconfigure the FPGA to perform the RNA alignment 410 and (ii) sends data representing the reference genome to the FPGA that is saved on D-RAM of the FPGA board or other high-speed storage media. Unlike the second FPGA invocation 432b to perform the DNA alignment 416, the multiomics sequencing system 106 sends data for the reference genome unique to the RNA alignment 410, such as a General Transfer Format (GTF) file comprising data representing nucleotide sequences encoding genes or exons, a Browser Extensible Data (BED) file that comprises a homology table that identifies paralogous genomic regions or otherwise boosts certain genomic regions comprising paralogous genes (e.g., for gene fusion), or a masking file that masks non-coding regions of the reference genome. As part of the second FPGA invocation 432b to perform the DNA alignment 416, by contrast, the multiomics sequencing system 106 (i) sends a bit stream encoding for a second alignment model to the FPGA to reconfigure the FPGA to perform the DNA alignment 416 and (ii) again sends data representing the reference genome to the FPGA that is saved on D-RAM of the FPGA board or other high-speed storage media. Unlike the first FPGA invocation 432a to perform the RNA alignment 410, in some embodiments, the multiomics sequencing system 106 sends data for the reference genome unique to the DNA alignment 416, such as a different masking file that masks targeted sequences of alternate haplotypes. As noted above, in some embodiments, the multiomics sequencing system 106 performs such FPGA invocations in a different order, such as by aligning genomic sequences with a reference genome in a first FPGA invocation and aligning transcriptomic sequences with the reference genome in a second FPGA invocation.

In addition to different configurable-processor invocations to align reads, as further shown in FIG. 4B, the multiomics sequencing system 106 performs joint cell filtering 434. To jointly filter cells, in some embodiments, the multiomics sequencing system 106 determines single-cell counts of UMI sequences per gene and single-cell counts of aligned genomic reads per accessible genomic region. Based on such single-cell counts per gene and per accessible genomic region, the multiomics sequencing system 106 determines summed single-cell counts of target nucleotide sequences-including summed counts of UMI sequences per candidate cell and summed counts of aligned genomic reads per candidate cell. For instance, the multiomics sequencing system 106 determines two dimensions for each candidate cell, that is, a first dimension for summed counts of UMI sequences and summed counts of aligned genomic reads.

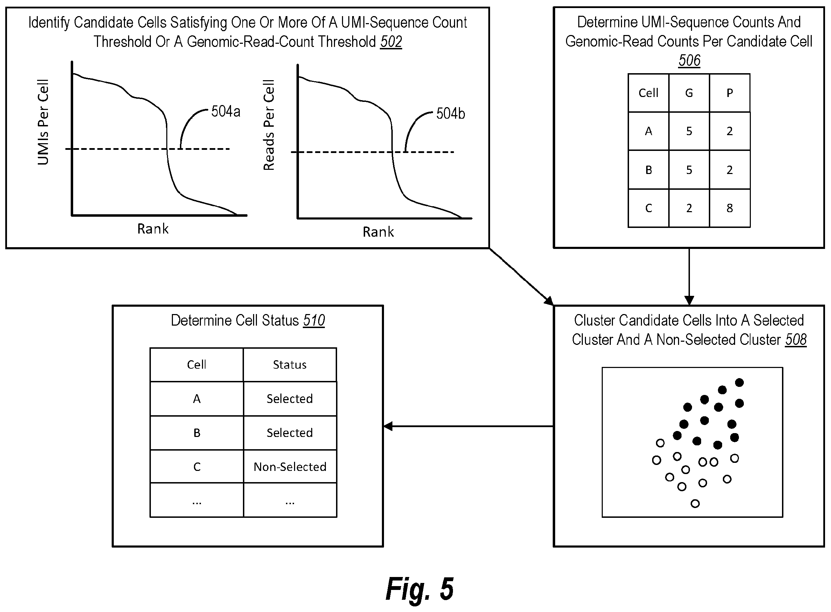

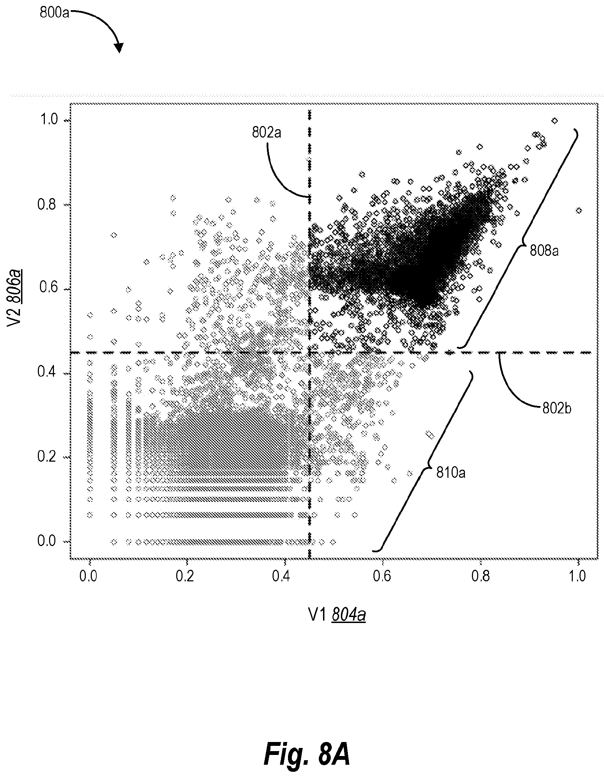

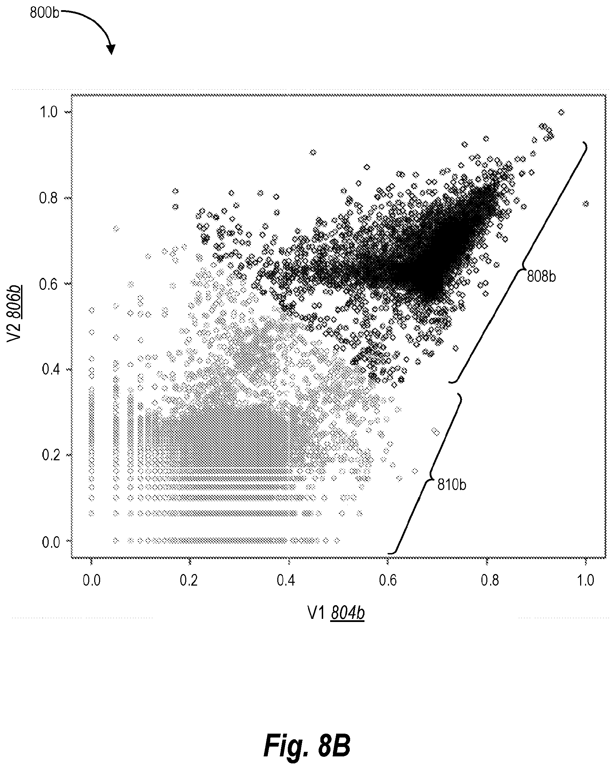

Having determined summed single-cell counts of target nucleotide sequences for each candidate cell, the multiomics sequencing system 106 clusters candidate cells based on respective combined single-cell counts of target nucleotide sequences. Based on a summed count of aligned transcriptomic reads (e.g., UMI sequences) per candidate cell and a summed count of aligned genomic reads, in some embodiments, the multiomics sequencing system 106 identifies a selected cluster of candidate cells and a non-selected cluster of candidate cells. As further explained below with respect to FIGS. 8A and 8B, in some cases, the multiomics sequencing system 106 applies a clustering model (e.g., K-means clustering) cluster candidate cells into the selected cluster of candidate cells and the non-selected cluster of candidate cells based on summed single-cell counts. If a candidate cell satisfies neither the threshold for summed counts of UMI sequences nor the threshold for summed counts of aligned genomic reads, in some implementations, the candidate cell is not part of the selected cluster of candidate cells. As described below, however, the multiomics sequencing system 106 can sometimes apply a clustering model that re-categorizes or re-clusters data points representing cells that fail to satisfy one of the threshold summed count of UMI sequences or the threshold summed count of aligned genomic reads into a selected cluster of candidate cells. Accordingly, in certain embodiments, the multiomics sequencing system 106 selects a subset of candidate cells as more likely representing real biological cells (or valid cells) of the sample when the selected subset of candidate cells satisfies both a threshold summed count of UMI sequences per candidate cell and a threshold summed count of aligned genomic reads. This disclosure describes joint cell filing further below with respect to FIGS. 5-8B.

As suggested above, by performing the joint cell filtering 434 and using a single multiomics executable file, the multiomics sequencing system 106 facilitates storing single-cell counts for aligned transcriptomic reads and aligned genomic reads per target nucleotide sequence, one or more intermediate matrices of summed single-cell counts, and other data on high-speed storage media, such as RAM of an FPGA board. As the multiomics sequencing system 106 determines either transcriptomic-read counts or genomic-read counts, the multiomics sequencing system 106 can retain such counts on RAM as it progresses to the joint cell filtering 434 without waiting for a separate cell-filtering process to conclude.

As further shown in FIG. 4B, the multiomics sequencing system 106 can quickly generate single-cell multiomics outputs 438 after the joint cell filtering 434. Because the multiomics sequencing system 106 determines single-cell counts of both UMI sequences and genomic reads for target nucleotide sequences and selects a subset of candidate cells, the multiomics sequencing system 106 can likewise generate data representing candidate cells with metrics for both a cell's transcriptome and genome. In particular, the multiomics sequencing system 106 generates a cell-by-feature matrix 436. In some embodiments, the cell-by-feature matrix 436 comprises both single-cell counts of aligned transcriptomic reads and single-cell counts of aligned genomic reads for target nucleotide sequences organized by each candidate cell within the selected subset of candidate cells. The cell-by-feature matrix 436 can accordingly be searched by candidate cell and provide a snapshot of different "omes" within a given cell.

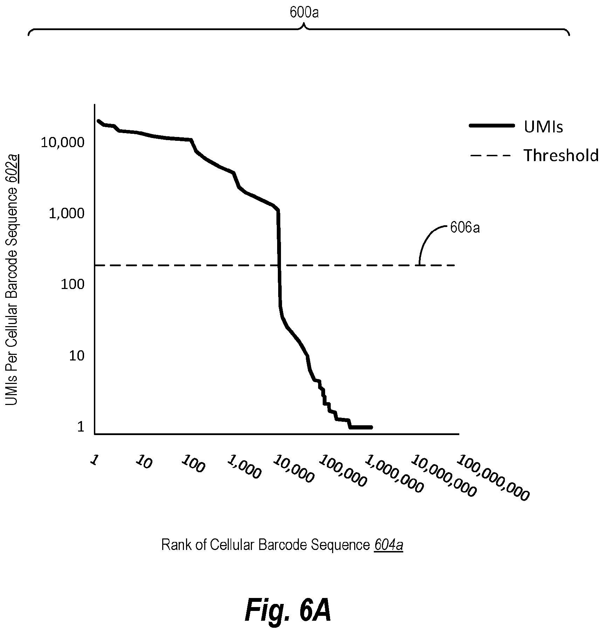

As just suggested, the multiomics sequencing system 106 can jointly filter candidate cells based on single-cell UMI-sequence counts and single-cell genomic-read counts. In accordance with one or more embodiments, FIG. 5 depicts an overview of the multiomics sequencing system 106 jointly filtering candidate cells by determining which candidate cells satisfy thresholds for single-cell UMI-sequence counts and single-cell read counts and clustering candidate cells according to a first dimension for UMI-sequence counts and a second dimension for genomic-read counts. The following paragraphs describe acts depicted in FIG. 5 and explained further in FIGS. 6A-8B performed or facilitated by the multiomics sequencing system 106 executing a single multiomics executable file.

As shown in FIG. 5, for instance, the multiomics sequencing system 106 identifies candidate cells satisfying one or more of a UMI-sequence-count threshold or a genomic-read-count threshold 502. In particular, the multiomics sequencing system 106 determines a single-cell UMI-sequence count and a single-cell genomic-read count for each cellular barcode sequence representing a candidate cell. The multiomics sequencing system 106 further identifies a threshold 504a for single-cell UMI-sequence counts corresponding to genes, such as a threshold single-cell UMI-sequence count corresponding to a precipitous decline or threshold difference in UMI-sequence counts. Similarly, the multiomics sequencing system 106 identifies a threshold 504b for single-cell genomic-read counts corresponding to accessible genomic regions, such as a threshold single-cell genomic-read count corresponding to a precipitous decline or threshold difference in genomic-read counts. As explained further below, in some embodiments, the multiomics sequencing system 106 uses the thresholds 504a and 504b to initially cluster candidate cells into an initially selected cluster of candidate cells and an initially non-selected cluster of candidate cells.

While this disclosure frequently refers to counts of UMI sequences and a UMI-sequence-count threshold and counts of genomic reads and a genomic-read-count threshold, in some embodiments, the multiomics sequencing system 106 may determine and use counts of aligned transcriptomic reads and a transcriptomic-read-count threshold and/or counts of nucleotide "read fragments" overlapping "peaks" or read-coverage peaks and a peak-fragment-count threshold.

In addition to determining the threshold 504a for single-cell UMI-sequence counts and the threshold 504b for single-cell genomic-read counts, the multiomics sequencing system 106 further determines UMI-sequence counts and genomic-read counts per candidate cell 506. For each candidate cell, for example, the multiomics sequencing system 106 determines a single-cell count of UMI sequences per gene and a single-cell count of genomic reads per accessible genomic region corresponding to a read-coverage peak. Based on such single-cell counts per gene and per accessible genomic region, the multiomics sequencing system 106 determines summed single-cell counts of UMI sequences for a set of genes within a candidate cell and summed single-cell counts of genomic reads for a set of accessible genomic regions within a candidate cell corresponding to read-coverage peaks.

As further shown in FIG. 5, the multiomics sequencing system 106 further clusters candidate cells into a selected cluster and a non-selected cluster 508 based on the summed single-cell counts. In some embodiments, for instance, the multiomics sequencing system 106 identifies a selected cluster of candidate cells satisfying both (i) a threshold summed count of UMI sequences and (ii) a threshold summed count of aligned genomic reads relative to a non-selected cluster of candidate cells, such as thresholds 504a and 504b. Based on the thresholds 504a and 504b, for instance, the multiomics sequencing system 106 optionally clusters candidate cells into an initially selected cluster of candidate cells and an initially non-selected cluster of candidate cells. In addition or in the alternative to such initial clustering, the multiomics sequencing system 106 applies a clustering model to cluster data points representing candidate cells according to a first dimension for summed counts of UMI sequences and a second dimension for summed counts of genomic reads.

In addition to clustering, as further shown in FIG. 5, the multiomics sequencing system 106 determines cell status 510 for each candidate cell. If a candidate cell is grouped together with the selected cluster of candidate cells, for example, the multiomics sequencing system 106 determines the candidate cell is selected and more likely to represent a real biological cell of a sample. But if a candidate cell is grouped together with the non-selected cluster of candidate cells, the multiomics sequencing system 106 determines the candidate cell is non-selected and less likely to represent a real biological cell of the sample.

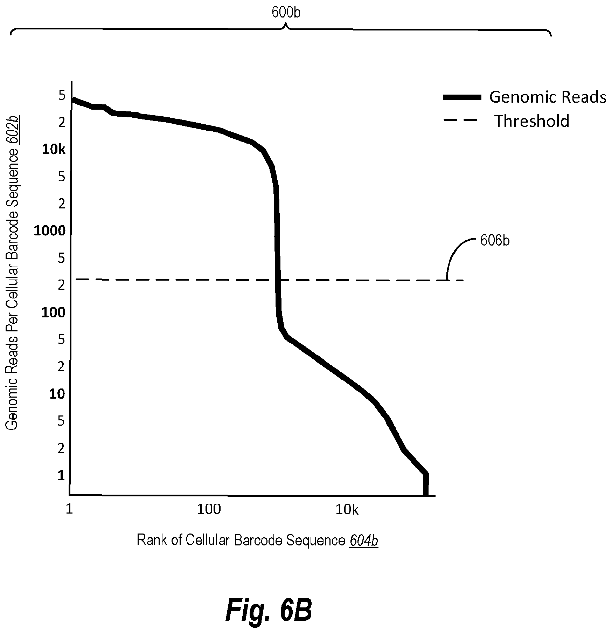

The following paragraphs describing FIGS. 6A-8B provide additional details concerning the joint cell filtering summarized in FIG. 5. In accordance with one or more embodiments, FIGS. 6A and 6B depict the multiomics sequencing system 106 using knee-plot graphs 600a and 600b to identify candidate cells that satisfy one or more of a UMI-sequence-count threshold or a genomic-read-count threshold. As an overview of processes suggested by the knee-plot graphs 600a and 600b, the multiomics sequencing system 106 (i) determines a count of UMI sequences and a count of genomic reads per cellular barcode sequence, (ii) ranks cellular barcode sequences according to their respective counts of UMI sequences and counts of genomic reads, and (iii) determines a UMI-sequence-count threshold and a genomic-read-count threshold, respectively. As explained with respect to FIGS. 8A and 8B below, in some embodiments, the multiomics sequencing system 106 uses the UMI-sequence-count threshold and the genomic-read-count threshold to initially cluster candidate cells into an initially selected cluster of candidate cells and an initially non-selected cluster of candidate cells.

As suggested by FIG. 6A, for instance, the multiomics sequencing system 106 determines a count of UMI sequences per cellular barcode sequence and ranks cellular barcode sequences according to a number of UMI sequences for each cellular barcode sequence. Based on fields or headers of a transcriptomic-read sequencing file (e.g., FASTQ), the multiomics sequencing system 106 counts a total number of UMI sequences per cellular barcode sequence representing a candidate cell. The multiomics sequencing system 106 further ranks cellular barcode sequences according to numbers of corresponding UMI sequences on a logarithmic scale. Accordingly, as shown in the knee-plot graph 600a, the y-axis represents UMIs per cellular barcode sequence 602a and the x-axis represents rank of cellular barcode sequence 604a according to numbers of corresponding UMI sequences.

As further suggested by FIG. 6A, the multiomics sequencing system 106 determines a UMI-sequence-count threshold 606a for filtering candidate cells. In particular, the multiomics sequencing system 106 executes a knee-plot algorithm to identify a precipitous decline or threshold difference in UMI-sequence counts among candidate cells. Indeed, in some cases, such a threshold is referred to as a "knee" or a false discovery rate (FDR) threshold. As shown in the knee-plot graph 600a, for instance, the multiomics sequencing system 106 determines the UMI-sequence-count threshold 606a as at approximately 200 UMI sequences. In some embodiments, the multiomics sequencing system 106 uses the UMI-sequence-count threshold 606a to initially cluster candidate cells into an initially selected cluster of candidate cells and an initially non-selected cluster of candidate cells, as described further below with respect to FIG. 8A.

As suggested by FIG. 6B, by contrast, the multiomics sequencing system 106 determines a count of aligned genomic reads (e.g., ATAC reads) per cellular barcode sequence and ranks cellular barcode sequences according to a number of genomic reads for each cellular barcode sequence. Based on fields or headers of a genomic-read sequencing file (e.g., FASTQ), the multiomics sequencing system 106 counts a total number of genomic reads per cellular barcode sequence representing a candidate cell. As suggested above, in some cases, the genomic reads are described or represented as nucleotide "read fragments" from genomic reads overlapping "peaks" or read-coverage peaks. The multiomics sequencing system 106 further ranks cellular barcode sequences according to numbers of corresponding genomic reads on a logarithmic scale. Accordingly, as shown in the knee-plot graph 600b, the y-axis represents genomic reads per cellular barcode sequence 602b and the x-axis represents rank of cellular barcode sequence 604b according to numbers of corresponding genomic reads.

As further suggested by FIG. 6B, the multiomics sequencing system 106 determines a genomic-read-count threshold 606b for filtering candidate cells. In particular, the multiomics sequencing system 106 executes a knee-plot algorithm to identify a precipitous decline or threshold difference in genomic-read counts among candidate cells. As shown in the knee-plot graph 600b, for instance, the multiomics sequencing system 106 determines the genomic-read-count threshold 606b as at approximately 200 or more genomic reads. In some embodiments, the multiomics sequencing system 106 uses the genomic-read-count threshold 606b to initially cluster candidate cells into an initially selected cluster of candidate cells and an initially non-selected cluster of candidate cells, as described further below with respect to FIG. 8A.

As noted above, in some embodiments, the multiomics sequencing system 106 identifies candidate cells that satisfies both the UMI-sequence-count threshold 606a and the genomic-read-count threshold 606b. By identifying candidate cells that satisfy one or both thresholds, the multiomics sequencing system 106 identifies cellular barcode sequences that more likely represent real biological cells (or valid cells) of a sample. In some cases, sample library fragments from which transcriptomic reads and genomic reads are synthesized are placed in droplets into some (but not all) wells of a nucleotide-sample slide (e.g., flow cell) to avoid cross contamination. Accordingly, some wells include droplets without target RNA or DNA from a sample, but may nevertheless include nucleotides with cellular barcode sequences or other non-targeted biomaterial. The UMI-sequence-count threshold 606a and the genomic-read-count threshold 606b provide bases upon which the multiomics sequencing system 106 distinguishes between empty droplets in wells or noise and wells comprising RNA or DNA from a sample.

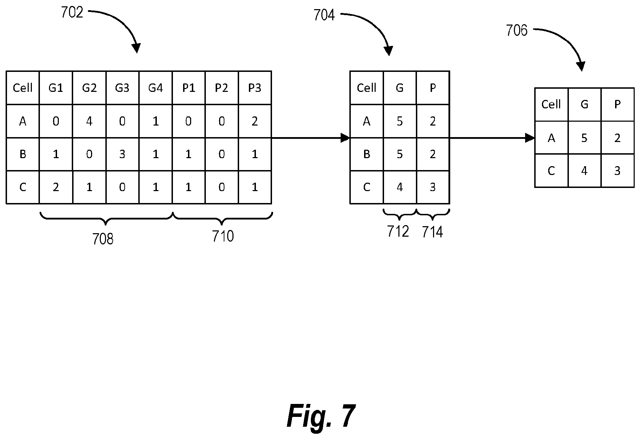

In addition to or after identifying candidate cells satisfying such count thresholds, in some embodiments, the multiomics sequencing system 106 determines UMI-sequence counts and genomic-read counts per candidate cell. In accordance with one or more embodiments, FIG. 7 depicts the multiomics sequencing system 106 determining UMI-sequence counts and genomic-read counts for each candidate cell f and deduplicating candidate cells. As an overview of processes suggested by FIG. 7, the multiomics sequencing system 106 (i) determines single-cell counts of UMI sequences and single-cell counts of genomic reads per target genomic sequence, (ii) determines summed single-cell counts of UMI sequences and summed single-cell counts of genomic reads for a set of target genomic sequences, and (iii) deduplicates candidate cells based on such summed single-cell counts. As suggested below, in some embodiments, the multiomics sequencing system 106 determines single-cells counts depicted in consolidated intermediate matrices 702, 704, and 706 and deduplicates candidate cells before applying a UMI-sequence-count threshold or a genomic-read-count threshold.

As shown in the consolidated intermediate matrix 702 of FIG. 7, for instance, the multiomics sequencing system 106 determines, for each candidate cell, single-cell counts 708 of UMI sequences per gene. In the consolidated intermediate matrix 702, for instance, the multiomics sequencing system 106 determines candidate cell A comprises zero UMI sequences for transcriptomic reads overlapping with a first gene (G1), four UMI sequences for transcriptomic reads overlapping with a second gene (G2), zero UMI sequences for transcriptomic reads overlapping with a third gene (G3), and one UMI sequence for transcriptomic reads overlapping with a fourth gene (G4).

As further shown in the consolidated intermediate matrix 702, the multiomics sequencing system 106 determines, for each candidate cell, single-cell counts 710 of genomic reads per accessible genomic region corresponding to a read-coverage peak. In the consolidated intermediate matrix 702, for instance, the multiomics sequencing system 106 determines candidate cell C comprises one genomic read overlapping with a first accessible genomic region corresponding to a read-coverage peak (P1), zero genomic reads overlapping with a second accessible genomic region corresponding to a read-coverage peak (P2), and one genomic read overlapping with a third accessible genomic region corresponding to a read-coverage peak (P3).

While FIG. 7 depicts candidate cells A, B, and C for illustrative purposes, in some embodiments, the multiomics sequencing system 106 represents candidate cells by cellular barcode sequences or an identifier that corresponds to different cellular barcode sequences representing a same candidate cell (e.g., a number, alphanumeric, or code for a candidate cell represented by one cellular barcode sequence for transcriptomic reads and another cellular barcode sequence for genomic reads). Indeed, the multiomics sequencing system 106 may generate a consolidated intermediate matrix representing tens of thousands, hundreds of thousands, or millions of candidate cells. Similarly, while FIG. 7 depicts four example genes and three example accessible genomic regions, in practice, the multiomics sequencing system 106 may generate a consolidated intermediate matrix representing hundreds, thousands, or millions of genes or accessible genomic regions.

After determining target-nucleotide-sequence-specific single-cell counts, as further shown in FIG. 7, the multiomics sequencing system 106 determines summed single-cell counts of UMI sequences and summed single-cell counts of genomic reads for all target genomic sequences. As shown in the consolidated intermediate matrix 704, for instance, the multiomics sequencing system 106 determines summed single-cell counts 712 of UMI sequences. In the consolidated intermediate matrix 704, for instance, the multiomics sequencing system 106 determines candidate cell A comprises a sum total of five UMI sequences for transcriptomic reads overlapping with either the first gene (G1), the second gene (G2), the third gene (G3), or the fourth gene (G4). As further shown in the consolidated intermediate matrix 704, the multiomics sequencing system 106 determines summed single-cell counts 714 of genomic reads. In the consolidated intermediate matrix 704, for instance, the multiomics sequencing system 106 determines cell C comprises a sum total of three genomic reads overlapping with either the first accessible genomic region corresponding to a read-coverage peak (P1), the second accessible genomic region corresponding to a read-coverage peak (P2), or the third accessible genomic region corresponding to a read-coverage peak (P3).

After determining summed single-cell counts, as further indicated by FIG. 7, the multiomics sequencing system 106 deduplicates certain candidate cells based on summed single-cell counts of UMI sequences and summed single-cell counts of genomic reads for all or a set of target genomic sequences. For example, in some embodiments, the multiomics sequencing system 106 deduplicates candidate cells exhibiting both a same summed single-cell count of UMI sequences and a same summed single-cell count of genomic reads. Alternatively, the multiomics sequencing system 106 deduplicates candidate cells exhibiting both a summed single-cell count of UMI sequences and a summed single-cell count of genomic reads within a threshold count difference (e.g., one count difference for summed UMI sequences or genomic reads). Regardless of whether an exact count match or a threshold count difference is implemented, in some cases, the multiomics sequencing system 106 uses a hash function to map a first dimension of summed single-cell count of UMI sequences and a second dimension of summed single-cell count of genomic reads to a hash representing a candidate cell. When candidate cells with different cellular barcode sequences are mapped to a same hash, one of the cellular barcode sequences are removed.

As shown by a transition from the consolidated intermediate matrix 704 to the consolidated intermediate matrix 706, for instance, the multiomics sequencing system 106 deduplicates candidate cell A and candidate cell B. Because both candidate cells A and B exhibit (i) a sum total of five UMI sequences for transcriptomic reads overlapping with G1-G4 and (ii) a sum total of two genomic reads overlapping with P1-P3, the multiomics sequencing system 106 removes candidate cell B. As shown in the consolidated intermediate matrix 706, the multiomics sequencing system 106 filters down the candidate cells A, B, and C to candidate cells A and C.

After determining summed single-cell counts of UMI sequences and genomic reads and deduplicating candidate cells, in some cases, the multiomics sequencing system 106 clusters candidate cells into a selected cluster and a non-selected cluster. In accordance with one or more embodiments, FIGS. 8A and 8B depict the multiomics sequencing system 106 using clustering models represented by cluster graphs 800a and 800b to cluster candidate cells based on summed single-cell counts of UMI sequences and genomic reads. In particular, FIG. 8A depicts the multiomics sequencing system 106 optionally clustering candidate cells into an initially selected cluster of candidate cells and an initially non-selected cluster of candidate cells based on thresholds for summed single-cell counts of UMI sequences and genomic reads. FIG. 8B depicts the multiomics sequencing system 106 using a clustering model to cluster candidate cells (or refine the initial clusters) into a selected cluster of candidate cells and a non-selected cluster of candidate cells based on summed single-cell counts of UMI sequences and genomic reads.

As shown in the cluster graph 800a in FIG. 8A, for instance, the multiomics sequencing system 106 clusters data points corresponding to cellular barcode sequences based on two dimensions. The first dimension represents summed counts of UMI sequences for each candidate cell. The second dimension represents summed counts of aligned genomic reads for each candidate cell. In some cases, the multiomics sequencing system 106 log normalizes the summed single-cell counts of UMI sequences and the summed single-cell counts of genomic reads. As shown in FIG. 8A, for instance, the x-axis of the cluster graph 800a represents log-normalized summed single-cell counts of UMI sequences 804a, and the y-axis of the cluster graph 800a represents log-normalized summed single-cell counts of genomic reads 806a.

As further indicated by the cluster graph 800a, the multiomics sequencing system 106 optionally clusters the data points corresponding to cellular barcode sequences based on a UMI-sequence-count threshold 802a and a genomic-read-count threshold 802b. The multiomics sequencing system 106 determines the UMI-sequence-count threshold 802a and the genomic-read-count threshold 802b by a performing knee-plot algorithm to identify a precipitous decline or threshold difference in UMI-sequence counts and genomic-read counts, respectively. Indeed, in some embodiments, the UMI-sequence-count threshold 802a and the genomic-read-count threshold 802b constitute the UMI-sequence-count threshold 606a and the genomic-read-count threshold 606b, respectively, described above with respect to FIGS. 6A and 6B.

Based on the UMI-sequence-count threshold 802a and the genomic-read-count threshold 802b, the multiomics sequencing system 106 clusters the data points corresponding to cellular barcode sequences into an initially selected cluster of candidate cells 808a and an initially non-selected cluster of candidate cells 810a. In particular, the multiomics sequencing system 106 clusters cellular barcode sequences satisfying only the UMI-sequence-count threshold 802a in a bottom-right quadrant of the cluster graph 800a, cellular barcode sequences that satisfy neither the UMI-sequence-count threshold 802a nor the genomic-read-count threshold 802b in a bottom-left quadrant of the cluster graph 800a, cellular barcode sequences that satisfy only the genomic-read-count threshold 802b in the top-left quadrant of the cluster graph 800a, and cellular barcode sequences that satisfy both the UMI-sequence-count threshold 802a and the genomic-read-count threshold 802b in the top-right quadrant of the cluster graph 800a. Indeed the cellular barcode sequences in the top-right quadrant constitute the initially selected cluster of candidate cells 808a, and the cellular barcode sequences in the other quadrants of the cluster graph 800a constitute the initially non-selected cluster of candidate cells 810a.

In addition or in the alternative to clustering based on thresholds for summed single-cell counts, as shown in FIG. 8B, the multiomics sequencing system 106 uses a clustering model to cluster candidate cells (or refine the initial clusters) into a selected cluster of candidate cells and a non-selected cluster of candidate cells based on summed single-cell counts. As shown in FIG. 8B, the x-axis of the cluster graph 800b represents log-normalized summed single-cell counts of UMI sequences 804b, and the y-axis of the cluster graph 800b represents log-normalized summed single-cell counts of genomic reads 806b. Based on summed single-cell counts of UMI sequences and summed single-cell counts of genomic reads, the multiomics sequencing system 106 executes a K-means clustering algorithm to cluster candidate cells into a selected cluster of candidate cells 808b and a non-selected cluster of candidate cells 810b. As shown by a comparison of the cluster graph 800a and the cluster graph 800b, K-means clustering captures cellular barcode sequences as part of the selected cluster of candidate cells 808b that would have otherwise been excluded based on the UMI-sequence-count threshold 802a or the genomic-read-count threshold 802b.

In the alternative to K-means clustering, the multiomics sequencing system 106 can use other suitable clustering models to cluster candidate cells into a selected cluster of candidate cells and a non-selected cluster of candidate cells based on summed single-cell counts. For instance, the multiomics sequencing system 106 can apply X-means clustering, Akaike information criterion, Bayesian information criterion, or another suitable method of determining clusters.

As indicated above, the multiomics sequencing system 106 can generate single-cell multiomics outputs for the selected cluster of candidate cells and/or the non-selected cluster of candidate cells. For example, in some embodiments, the multiomics sequencing system 106 generates a joint cell-by-feature matrix comprising both single-cell counts of UMI sequences and single-cell counts of aligned genomic reads for target nucleotide sequences organized by each candidate cell within the selected subset or cluster of candidate cells. Alternatively, the multiomics sequencing system 106 generates a joint cell-by-feature matrix comprising both single-cell counts of UMI sequences and single-cell counts of aligned genomic reads for target nucleotide sequences organized by each candidate cell within both the selected subset or cluster of candidate cells and the non-selected subset or cluster of candidate cells.

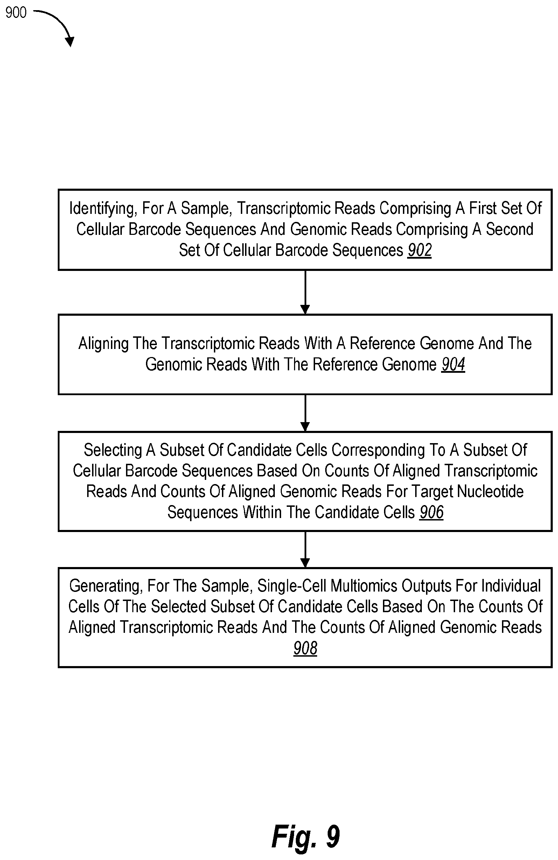

Turning now to FIG. 9, this figure illustrates a flowchart of a series of acts 900 of performing a single-cell multiomics analysis in accordance with one or more embodiments of the present disclosure. While FIG. 9 illustrates acts according to one embodiment, alternative embodiments may omit, add to, reorder, and/or modify any of the acts shown in FIG. 9. The acts of FIG. 9 can be performed as part of a method. Alternatively, a non-transitory computer readable storage medium can comprise instructions that, when executed by one or more processors, cause a computing device or a system to perform the acts depicted in FIG. 9. In still further embodiments, a system comprising at least one processor and a non-transitory computer readable medium comprising instructions that, when executed by one or more processors, cause the system to perform the acts of FIG. 9. In some cases, the at least one processor comprises a configurable processor and executing the at least one processor comprises configuring the configurable processor.

As shown in FIG. 9, the acts 900 include an act 902 of identifying, for a sample, transcriptomic reads comprising a first set of cellular barcode sequences and genomic reads comprising a second set of cellular barcode sequences. In particular, in some embodiments, the act 902 includes identifying, for a sample and utilizing a multiomics executable file, transcriptomic reads comprising a first set of cellular barcode sequences representing candidate cells and genomic reads comprising a second set of cellular barcode sequences representing candidate cells.

As suggested above, in some cases, the first set of cellular barcode sequences differs from the second set of cellular barcode sequences, and the first set of cellular barcode sequences and the second set of cellular barcode sequences correspond to a same set of candidate cells. Further, in certain embodiments, the transcriptomic reads comprise a sequence of complementary DNA synthesized from single-stranded ribonucleic acid (RNA) from the sample; and the genomic reads comprise a nucleotide sequence of genomic deoxyribonucleic acid (DNA) complementing a genomic sequence from the sample. Additionally, or alternatively, in certain cases, the genomic reads comprise Assay for Transposase-Accessible Chromatin (ATAC) reads for the sample.

As further shown in FIG. 9, the acts 900 include an act 904 of aligning the transcriptomic reads with a reference genome and the genomic reads with the reference genome. In particular, in certain implementations, the act 904 includes aligning, utilizing the multiomics executable file, the transcriptomic reads with a reference genome and the genomic reads with the reference genome.

For example, in some cases, aligning the transcriptomic reads and the genomic reads with the reference genome comprises: configuring a configurable processor to execute a first alignment model that aligns the transcriptomic reads with the reference genome; and configuring the configurable processor to execute a second alignment model that aligns the genomic reads with the reference genome. Similarly, in certain cases, aligning the transcriptomic reads and the genomic reads with the reference genome comprises: configuring a configurable processor to execute a first alignment model that aligns the genomic reads with the reference genome; and configuring the configurable processor to execute a second alignment model that aligns the transcriptomic reads with the reference genome.

As further shown in FIG. 9, the acts 900 include an act 906 of selecting a subset of candidate cells corresponding to a subset of cellular barcode sequences based on counts of aligned transcriptomic reads and counts of aligned genomic reads for target nucleotide sequences within the candidate cells. In particular, in certain implementations, the act 906 includes selecting, utilizing the multiomics executable file, a subset of candidate cells corresponding to a subset of cellular barcode sequences based on counts of aligned transcriptomic reads and counts of aligned genomic reads for target nucleotide sequences within the candidate cells.

For example, in some cases, selecting the subset of candidate cells corresponding to the subset of cellular barcode sequences comprises: determining, for each target nucleotide sequence within each candidate cell, a first count of aligned transcriptomic reads and a second count of aligned genomic reads; and clustering, from the first set of cellular barcode sequences and the second set of cellular barcode sequences, cellular barcode sequences in a selected cluster of candidate cells and a non-selected cluster of candidate cells based on the first count of aligned transcriptomic reads and the second count of aligned genomic reads for each target nucleotide sequence within each candidate cell.

Relatedly, in certain embodiments, determining the first count of aligned transcriptomic reads comprises determining, for each gene encoded by a nucleotide sequence within each candidate cell, a count of unique molecular identifier (UMI) sequences corresponding to aligned genomic reads; and determining the second count of aligned genomic reads comprises determining, for each accessible genomic region corresponding to a read-coverage peak within each candidate cell, a count of read fragments from aligned genomic reads.

Further, in some implementations, clustering the cellular barcode sequences comprises clustering the cellular barcode sequences into the selected cluster of candidate cells and a non-selected cluster of candidate cells based on a first dimension for summed counts of aligned transcriptomic reads for each candidate cell and a second dimension for summed counts of aligned genomic reads for each candidate cell.

Additionally, or alternatively, in certain embodiments, determining that an initial subset of candidate cells satisfies a threshold for counts of aligned transcriptomic reads and a threshold for counts of aligned genomic reads; determining the first count of aligned transcriptomic reads for each threshold-passing candidate cell from the initial subset of candidate cells and the second count of aligned genomic reads for each threshold-passing candidate cell from the initial subset of candidate cells; and clustering the cellular barcode sequences corresponding to the initial subset of candidate cells in the selected cluster of candidate cells and the non-selected cluster of candidate cells.

As noted above, in some cases, selecting the subset of candidate cells corresponding to the subset of cellular barcode sequences comprises: storing, on random-access memory, the counts of aligned transcriptomic reads and the counts of aligned genomic reads for the target nucleotide sequences; and selecting the subset of candidate cells corresponding to the subset of cellular barcode sequences based on the counts of aligned transcriptomic reads and the counts of aligned genomic reads stored on the random-access memory.

As further shown in FIG. 9, the acts 900 include an act 908 of generating, for the sample, single-cell multiomics outputs for individual cells of the selected subset of candidate cells based on the counts of aligned transcriptomic reads and the counts of aligned genomic reads. In particular, in certain implementations, the act 908 includes generating, for the sample and utilizing the multiomics executable file, single-cell multiomics outputs for individual cells of the selected subset of candidate cells based on the counts of aligned transcriptomic reads and the counts of aligned genomic reads.

As suggested above, in some cases, generating the single-cell multiomics outputs for individual cells comprises generating a joint cell-by-feature matrix comprising both single-cell counts of aligned transcriptomic reads and single-cell counts of aligned genomic reads for target nucleotide sequences organized by each candidate cell within the selected subset of candidate cells. Additionally, or alternatively, in certain embodiments, generating the single-cell multiomics outputs for individual cells comprises: generating a first set of single-cell metrics indicating gene expression for each candidate cell of the selected subset of candidate cells based on the counts of aligned transcriptomic reads; and generating a second set of single-cell metrics indicating accessible genomic deoxyribonucleic acid (DNA) corresponding to open chromatin for each candidate cell of the selected subset of candidate cells based on the counts of aligned genomic reads.

The methods described herein can be used in conjunction with a variety of nucleic acid sequencing techniques. Particularly applicable techniques are those wherein nucleic acids are attached at fixed locations in an array such that their relative positions do not change and wherein the array is repeatedly imaged. Embodiments in which images are obtained in different color channels, for example, coinciding with different labels used to distinguish one nucleobase type from another are particularly applicable. In some embodiments, the process to determine the nucleotide sequence of a target nucleic acid (i.e., a nucleic-acid polymer) can be an automated process. Preferred embodiments include sequencing-by-synthesis (SBS) techniques.

SBS techniques generally involve the enzymatic extension of a nascent nucleic acid strand through the iterative addition of nucleotides against a template strand. In traditional methods of SBS, a single nucleotide monomer may be provided to a target nucleotide in the presence of a polymerase in each delivery. However, in the methods described herein, more than one type of nucleotide monomer can be provided to a target nucleic acid in the presence of a polymerase in a delivery.

SBS can utilize nucleotide monomers that have a terminator moiety or those that lack any terminator moieties. Methods utilizing nucleotide monomers lacking terminators include, for example, pyrosequencing and sequencing using 7-phosphate-labeled nucleotides, as set forth in further detail below. In methods using nucleotide monomers lacking terminators, the number of nucleotides added in each cycle is generally variable and dependent upon the template sequence and the mode of nucleotide delivery. For SBS techniques that utilize nucleotide monomers having a terminator moiety, the terminator can be effectively irreversible under the sequencing conditions used as is the case for traditional Sanger sequencing which utilizes dideoxynucleotides, or the terminator can be reversible as is the case for sequencing methods developed by Solexa (now Illumina, Inc.).

SBS techniques can utilize nucleotide monomers that have a label moiety or those that lack a label moiety. Accordingly, incorporation events can be detected based on a characteristic of the label, such as fluorescence of the label; a characteristic of the nucleotide monomer such as molecular weight or charge; a byproduct of incorporation of the nucleotide, such as release of pyrophosphate; or the like. In embodiments, where two or more different nucleotides are present in a sequencing reagent, the different nucleotides can be distinguishable from each other, or alternatively, the two or more different labels can be the indistinguishable under the detection techniques being used. For example, the different nucleotides present in a sequencing reagent can have different labels and they can be distinguished using appropriate optics as exemplified by the sequencing methods developed by Solexa (now Illumina, Inc.).

Preferred embodiments include pyrosequencing techniques. Pyrosequencing detects the release of inorganic pyrophosphate (PPi) as particular nucleotides are incorporated into the nascent strand (Ronaghi, M., Karamohamed, S., Pettersson, B., Uhlen, M. and Nyren, P. (1996) "Real-time DNA sequencing using detection of pyrophosphate release." Analytical Biochemistry 242(1), 84-9; Ronaghi, M. (2001) "Pyrosequencing sheds light on DNA sequencing." Genome Res. 11(1), 3-11; Ronaghi, M., Uhlen, M. and Nyren, P. (1998) "A sequencing method based on real-time pyrophosphate." Science 281(5375), 363; U.S. Pat. No. 6,210,891; U.S. Pat. No. 6,258,568 and U.S. Pat. No. 6,274,320. In pyrosequencing, released PPi can be detected by being immediately converted to adenosine triphosphate (ATP) by ATP sulfurylase, and the level of ATP generated is detected via luciferase-produced photons. The nucleic acids to be sequenced can be attached to features in an array and the array can be imaged to capture the chemiluminescent signals that are produced due to incorporation of a nucleotides at the features of the array. An image can be obtained after the array is treated with a particular nucleotide type (e.g., A, T, C or G). Images obtained after addition of each nucleotide type
will differ with regard to which features in the array are detected. These differences in the image reflect the different sequence content of the features on the array. However, the relative locations of each feature will remain unchanged in the images. The images can be stored, processed and analyzed using the methods set forth herein. For example, images obtained after treatment of the array with each different nucleotide type can be handled in the same way as exemplified herein for images obtained from different detection channels for reversible terminator-based sequencing methods.

In another exemplary type of SBS, cycle sequencing is accomplished by stepwise addition of reversible terminator nucleotides containing, for example, a cleavable or photobleachable dye label as described, for example, in WO 04/018497 and U.S. Pat. No. 7,057,026. This approach is being commercialized by Solexa (now Illumina Inc.), and is also described in WO 91/06678 and WO 07/123,744. The availability of fluorescently-labeled terminators in which both the termination can be reversed and the fluorescent label cleaved facilitates efficient cyclic reversible termination (CRT) sequencing. Polymerases can also be co-engineered to efficiently incorporate and extend from these modified nucleotides.

Preferably in reversible terminator-based sequencing embodiments, the labels do not substantially inhibit extension under SBS reaction conditions. However, the detection labels can be removable, for example, by cleavage or degradation. Images can be captured following incorporation of labels into arrayed nucleic acid features. In particular embodiments, each cycle involves simultaneous delivery of four different nucleotide types to the array and each nucleotide type has a spectrally distinct label. Four images can then be obtained, each using a detection channel that is selective for one of the four different labels. Alternatively, different nucleotide types can be added sequentially and an image of the array can be obtained between each addition step. In such embodiments, each image will show nucleic acid features that have incorporated nucleotides of a particular type. Different features are present or absent in the different images due the different sequence content of each feature. However, the relative position of the features will remain unchanged in the images. Images obtained from such reversible terminator-SBS methods can be stored, processed and analyzed as set forth herein. Following the image capture step, labels can be removed and reversible terminator moieties can be removed for subsequent cycles of nucleotide addition and detection. Removal of the labels after they have been detected in a particular cycle and prior to a subsequent cycle can provide the advantage of reducing background signal and crosstalk between cycles. Examples of useful labels and removal methods are set forth below.

In particular embodiments some or all of the nucleotide monomers can include reversible terminators. In such embodiments, reversible terminators/cleavable fluors can include fluor linked to the ribose moiety via a 3' ester linkage (Metzker, Genome Res. 15:1767-1776 (2005)). Other approaches have separated the terminator chemistry from the cleavage of the fluorescence label (Ruparel et al., Proc Natl Acad Sci USA 102: 5932-7 (2005)). Ruparel et al described the development of reversible terminators that used a small 3' allyl group to block extension, but could easily be deblocked by a short treatment with a palladium catalyst. The fluorophore was attached to the base via a photocleavable linker that could easily be cleaved by a 30 second exposure to long wavelength UV light. Thus, either disulfide reduction or photocleavage can be used as a cleavable linker. Another approach to reversible termination is the use of natural termination that ensues after placement of a bulky dye on a dNTP. The presence of a charged bulky dye on the dNTP can act as an effective terminator through steric and/or electrostatic hindrance. The presence of one incorporation event prevents further incorporations unless the dye is removed. Cleavage of the dye removes the fluor and effectively reverses the termination. Examples of modified nucleotides are also described in U.S. Pat. No. 7,427,673, and U.S. Pat. No. 7,057,026.

Additional exemplary SBS systems and methods which can be utilized with the methods and systems described herein are described in U.S. Patent Application Publication No. 2007/0166705, U.S. Patent Application Publication No. 2006/0188901, U.S. Pat. No. 7,057,026, U.S. Patent Application Publication No. 2006/0240439, U.S. Patent Application Publication No. 2006/0281109, PCT Publication No. WO 05/065814, U.S. Patent Application Publication No. 2005/0100900, PCT Publication No. WO 06/064199, PCT Publication No. WO 07/010,251, U.S. Patent Application Publication No. 2012/0270305 and U.S. Patent Application Publication No. 2013/0260372.

Some embodiments can utilize detection of four different nucleotides using fewer than four different labels. For example, SBS can be performed utilizing methods and systems described in the incorporated materials of U.S. Patent Application Publication No. 2013/0079232. As a first example, a pair of nucleotide types can be detected at the same wavelength, but distinguished based on a difference in intensity for one member of the pair compared to the other, or based on a change to one member of the pair (e.g. via chemical modification, photochemical modification or physical modification) that causes apparent signal to appear or disappear compared to the signal detected for the other member of the pair. As a second example, three of four different nucleotide types can be
detected under particular conditions while a fourth nucleotide type lacks a label that is detectable under those conditions, or is minimally detected under those conditions (e.g., minimal detection due to background fluorescence, etc.). Incorporation of the first three nucleotide types into a nucleic acid can be determined based on presence of their respective signals and incorporation of the fourth nucleotide type into the nucleic acid can be determined based on absence or minimal detection of any signal. As a third example, one nucleotide type can include label(s) that are detected in two different channels, whereas other nucleotide types are detected in no more than one of the channels. The aforementioned three exemplary configurations are not considered mutually exclusive and can be used in various combinations. An exemplary embodiment that combines all three examples, is a fluorescent-based SBS method that uses a first nucleotide type that is detected in a first channel (e.g. dATP having a label that is detected in the first channel when excited by a first excitation wavelength), a second nucleotide type that is detected in a second channel (e.g. dCTP having a label that is detected in the second channel when excited by a second excitation wavelength), a third nucleotide type that is detected in both the first and the second channel (e.g. dTTP having at least one label that is detected in both channels when excited by the first and/or second excitation wavelength) and a fourth nucleotide type that lacks a label that is not, or minimally, detected in either channel (e.g. dGTP having no label).

Further, as described in the incorporated materials of U.S. Patent Application Publication No. 2013/0079232, sequencing data can be obtained using a single channel. In such so-called one-dye sequencing approaches, the first nucleotide type is labeled but the label is removed after the first image is generated, and the second nucleotide type is labeled only after a first image is generated. The third nucleotide type retains its label in both the first and second images, and the fourth nucleotide type remains unlabeled in both images.

Some embodiments can utilize sequencing by ligation techniques. Such techniques utilize DNA ligase to incorporate oligonucleotides and identify the incorporation of such oligonucleotides. The oligonucleotides typically have different labels that are correlated with the identity of a particular nucleotide in a sequence to which the oligonucleotides hybridize. As with other SBS methods, images can be obtained following treatment of an array of nucleic acid features with the labeled sequencing reagents. Each image will show nucleic acid features that have incorporated labels of a particular type. Different features are present or absent in the different images due the different sequence content of each feature, but the relative position of the features will remain unchanged in the images. Images obtained from ligation-based sequencing methods can be stored, processed and analyzed as set forth herein. Exemplary SBS systems and methods which can be utilized with the methods and systems described herein are described in U.S. Pat. No. 6,969,488, U.S. Pat. No. 6,172,218, and U.S. Pat. No. 6,306,597.

Some embodiments can utilize nanopore sequencing (Deamer, D. W. & Akeson, M. "Nanopores and nucleic acids: prospects for ultrarapid sequencing." Trends Biotechnol. 18, 147-151 (2000); Deamer, D. and D. Branton, "Characterization of nucleic acids by nanopore analysis". Acc. Chem. Res. 35:817-825 (2002); Li, J., M. Gershow, D. Stein, E. Brandin, and J. A. Golovchenko, "DNA molecules and configurations in a solid-state nanopore microscope" Nat. Mater. 2:611-615 (2003)). In such embodiments, the target nucleic acid passes through a nanopore. The nanopore can be a synthetic pore or biological membrane protein, such as a-hemolysin. As the target nucleic acid passes through the nanopore, each base-pair can be identified by measuring fluctuations in the electrical conductance of the pore. (U.S. Pat. No. 7,001,792; Soni, G. V. & Meller, "A. Progress toward ultrafast DNA sequencing using solid-state nanopores." Clin. Chem. 53, 1996-2001 (2007); Healy, K. "Nanopore-based single-molecule DNA analysis." Nanomed. 2, 459-481 (2007); Cockroft, S. L., Chu, J., Amorin, M. & Ghadiri, M. R. "A single-molecule nanopore device detects DNA polymerase activity with single-nucleotide resolution." J. Am. Chem. Soc. 130, 818-820 (2008)). Data obtained from nanopore sequencing can be stored, processed and analyzed as set forth herein. In particular, the data can be treated as an image in accordance with the exemplary treatment of optical images and other images that is set forth herein.

Some embodiments can utilize methods involving the real-time monitoring of DNA polymerase activity. Nucleotide incorporations can be detected through fluorescence resonance energy transfer (FRET) interactions between a fluorophore-bearing polymerase and y-phosphate-labeled nucleotides as described, for example, in U.S. Pat. No. 7,329,492 and U.S. Pat. No. 7,211,414 or nucleotide incorporations can be detected with zero-mode waveguides as described, for example, in U.S. Pat. No. 7,315,019 and using fluorescent nucleotide analogs and engineered polymerases as described, for example, in U.S. Pat. No. 7,405,281 and U.S. Patent Application Publication No. 2008/0108082. The illumination can be restricted to a zeptoliter-scale volume around a surface-tethered polymerase such that incorporation of fluorescently labeled nucleotides can be observed with low background (Levene, M. J. et al. "Zero-mode waveguides for single-molecule analysis at high concentrations." Science 299, 682-686 (2003); Lundquist, P. M. et al. "Parallel confocal detection of single molecules in real time." Opt. Left. 33, 1026-1028 (2008); Korlach, J. et al. "Selective aluminum passivation for targeted immobilization of single DNA polymerase molecules in zero-mode waveguide nano structures." Proc. Natl. Acad. Sci. USA 105, 1176-1181 (2008). Images obtained from such methods can be stored, processed and analyzed as set forth herein.

Some SBS embodiments include detection of a proton released upon incorporation of a nucleotide into an extension product. For example, sequencing based on detection of released protons can use an electrical detector and associated techniques that are commercially available from Ion Torrent (Guilford, CT, a Life Technologies subsidiary) or sequencing methods and systems described in US 2009/0026082 A1; US 2009/0127589 A1; US 2010/0137143 A1; or US 2010/0282617 A1. Methods set forth herein for amplifying target nucleic acids using kinetic exclusion can be readily applied to substrates used for detecting protons. More specifically, methods set forth herein can be used to produce clonal populations of amplicons that are used to detect protons.

The above SBS methods can be advantageously carried out in multiplex formats such that multiple different target nucleic acids are manipulated simultaneously. In particular embodiments, different target nucleic acids can be treated in a common reaction vessel or on a surface of a particular substrate. This allows convenient delivery of sequencing reagents, removal of unreacted reagents and detection of incorporation events in a multiplex manner. In embodiments using surface-bound target nucleic acids, the target nucleic acids can be in an array format. In an array format, the target nucleic acids can be typically bound to a surface in a spatially distinguishable manner. The target nucleic acids can be bound by direct covalent attachment, attachment to a bead or other particle or binding to a polymerase or other molecule that is attached to the surface. The array can include a single copy of a target nucleic acid at each site (also referred to as a feature) or multiple copies having the same sequence can be present at each site or feature. Multiple copies can be produced by amplification methods such as, bridge amplification or emulsion PCR as described in further detail below.

The methods set forth herein can use arrays having features at any of a variety of densities including, for example, at least about 10 features/cm2, 100 features/cm2, 500 features/cm2, 1,000 features/cm2, 5,000 features/cm2, 10,000 features/cm2, 50,000 features/cm2, 100,000 features/cm2, 1,000,000 features/cm2, 5,000,000 features/cm2, or higher.

An advantage of the methods set forth herein is that they provide for rapid and efficient detection of a plurality of target nucleic acid in parallel. Accordingly the present disclosure provides integrated systems capable of preparing and detecting nucleic acids using techniques known in the art such as those exemplified above. Thus, an integrated system of the present disclosure can include fluidic components capable of delivering amplification reagents and/or sequencing reagents to one or more immobilized DNA fragments, the system comprising components such as pumps, valves, reservoirs, fluidic lines and the like. A flow cell can be configured and/or used in an integrated system for detection of target nucleic acids. Exemplary flow cells are described, for example, in US 2010/0111768 A1 and US Ser. No. 13/273,666. As exemplified for flow cells, one or more of the fluidic components of an integrated system can be used for an amplification method and for a detection method. Taking a nucleic acid sequencing embodiment as an example, one or more of the fluidic components of an integrated system can be used for an amplification method set forth herein and for the delivery of sequencing reagents in a sequencing method such as those exemplified above. Alternatively, an integrated system can include separate fluidic systems to carry out amplification methods and to carry out detection methods. Examples of integrated sequencing systems that are capable of creating amplified nucleic acids and also determining the sequence of the nucleic acids include, without limitation, the MiSeqTM platform (Illumina, Inc., San Diego, CA) and devices described in US Ser. No. 13/273,666.

The sequencing system described above sequences nucleic-acid polymers present in samples received by a sequencing device. As described herein and defined above, a "sample" and its derivatives, is used in its broadest sense and includes any specimen, culture and the like that is suspected of including a target. In some embodiments, the sample comprises DNA, RNA, PNA, LNA, chimeric or hybrid forms of nucleic acids. The sample can include any biological, clinical, surgical, agricultural, atmospheric or aquatic-based specimen containing one or more nucleic acids. The term also includes any isolated nucleic acid sample such a genomic DNA, fresh-frozen or formalin-fixed paraffin-embedded nucleic acid specimen. It is also envisioned that the sample can be from a single individual, a collection of nucleic acid samples from genetically related members, nucleic acid samples from genetically unrelated members, nucleic acid samples (matched) from a single individual such as a tumor sample and normal tissue sample, or sample from a single source that contains two distinct forms of genetic material such as maternal and fetal DNA obtained from a maternal subject, or the presence of contaminating bacterial DNA in a sample that contains plant or animal DNA. In some embodiments, the source of nucleic acid material can include nucleic acids obtained from a newborn, for example as typically used for newborn screening.

The nucleic acid sample can include high molecular weight material such as genomic DNA (gDNA). The sample can include low molecular weight material such as nucleic acid molecules obtained from FFPE or archived DNA samples. In another embodiment, low molecular weight material includes enzymatically or mechanically fragmented DNA. The sample can include cell-free
circulating DNA. In some embodiments, the sample can include nucleic acid molecules obtained from biopsies, tumors, scrapings, swabs, blood, mucus, urine, plasma, semen, hair, laser capture microdissections, surgical resections, and other clinical or laboratory obtained samples. In some embodiments, the sample can be an epidemiological, agricultural, forensic or pathogenic sample. In some embodiments, the sample can include nucleic acid molecules obtained from an animal such as a human or mammalian source. In another embodiment, the sample can include nucleic acid molecules obtained from a non-mammalian source such as a plant, bacteria, virus or fungus. In some embodiments, the source of the nucleic acid molecules may be an archived or extinct sample or species.

Further, the methods and compositions disclosed herein may be useful to amplify a nucleic acid sample having low-quality nucleic acid molecules, such as degraded and/or fragmented genomic DNA from a forensic sample. In one embodiment, forensic samples can include nucleic acids obtained from a crime scene, nucleic acids obtained from a missing persons DNA database, nucleic acids obtained from a laboratory associated with a forensic investigation or include forensic samples obtained by law enforcement agencies, one or more military services or any such personnel. The nucleic acid sample may be a purified sample or a crude DNA containing lysate, for example derived from a buccal swab, paper, fabric or other substrate that may be impregnated with saliva, blood, or other bodily fluids. As such, in some embodiments, the nucleic acid sample may comprise low amounts of, or fragmented portions of DNA, such as genomic DNA. In some embodiments, target sequences can be present in one or more bodily fluids including but not limited to, blood, sputum, plasma, semen, urine and serum. In some embodiments, target sequences can be obtained from hair, skin, tissue samples, autopsy or remains of a victim. In some embodiments, nucleic acids including one or more target sequences can be obtained from a deceased animal or human. In some embodiments, target sequences can include nucleic acids obtained from non-human DNA such a microbial, plant or entomological DNA. In some embodiments, target sequences or amplified target sequences are directed to purposes of human identification. In some embodiments, the disclosure relates generally to methods for identifying characteristics of a forensic sample. In some embodiments, the disclosure relates generally to human identification methods using one or more target specific primers disclosed herein or one or more target specific primers designed using the primer design criteria outlined herein. In one embodiment, a forensic or human identification sample containing at least one target sequence can be amplified using any one or more of the target-specific primers disclosed herein or using the primer criteria outlined herein.

The components of the multiomics sequencing system 106 can include software, hardware, or both. For example, the components of the multiomics sequencing system 106 can include one or more instructions stored on a computer-readable storage medium and executable by processors of one or more computing devices (e.g., the client device 114). When executed by the one or more processors, the computer-executable instructions of the multiomics sequencing system 106 can cause the computing devices to perform the bubble detection methods described herein. Alternatively, the components of the multiomics sequencing system 106 can comprise hardware, such as special purpose processing devices to perform a certain function or group of functions. Additionally, or alternatively, the components of the multiomics sequencing system 106 can include a combination of computer-executable instructions and hardware.

Furthermore, the components of the multiomics sequencing system 106 performing the functions described herein with respect to the multiomics sequencing system 106 may, for example, be implemented as part of a stand-alone application, as a module of an application, as a plug-in for applications, as a library function or functions that may be called by other applications, and/or as a cloud-computing model. Thus, components of the multiomics sequencing system 106 may be implemented as part of a stand-alone application on a personal computing device or a mobile device. Additionally, or alternatively, the components of the multiomics sequencing system 106 may be implemented in any application that provides sequencing services including, but not limited to Illumina BaseSpace, Illumina DRAGEN, Illumina NextSeq, Illumina TruSeq, or Illumina TruSight software. "Illumina," "BaseSpace," "DRAGEN," "NextSeq," "TruSeq," and "TruSight," are either registered trademarks or trademarks of Illumina, Inc. in the United States and/or other countries.

Embodiments of the present disclosure may comprise or utilize a special purpose or general-purpose computer including computer hardware, such as, for example, one or more processors and system memory, as discussed in greater detail below. Embodiments within the scope of the present disclosure also include physical and other computer-readable media for carrying or storing computer-executable instructions and/or data structures. In particular, one or more of the processes described herein may be implemented at least in part as instructions embodied in a non-transitory computer-readable medium and executable by one or more computing devices (e.g., any of the media content access devices described herein). In general, a processor (e.g., a microprocessor) receives instructions, from a non-transitory computer-readable medium, (e.g., a memory, etc.), and executes those instructions, thereby performing one or more processes, including one or more of the processes described herein.

Computer-readable media can be any available media that can be accessed by a general purpose or special purpose computer system. Computer-readable media that store computer-executable instructions are non-transitory computer-readable storage media (devices). Computer-readable media that carry computer-executable instructions are transmission media. Thus, by way of example, and not limitation, embodiments of the disclosure can comprise at least two distinctly different kinds of computer-readable media: non-transitory computer-readable storage media (devices) and transmission media.

Non-transitory computer-readable storage media (devices) includes RAM, ROM, EEPROM, CD-ROM, solid state drives (SSDs) (e.g., based on RAM), Flash memory, phase-change memory (PCM), other types of memory, other optical disk storage, magnetic disk storage or other magnetic storage devices, or any other medium which can be used to store desired program code means in the form of computer-executable instructions or data structures and which can be accessed by a general purpose or special purpose computer.

A "network" is defined as one or more data links that enable the transport of electronic data between computer systems and/or modules and/or other electronic devices. When information is transferred or provided over a network or another communications connection (either hardwired, wireless, or a combination of hardwired or wireless) to a computer, the computer properly views the connection as a transmission medium. Transmissions media can include a network and/or data links which can be used to carry desired program code means in the form of computer-executable instructions or data structures and which can be accessed by a general purpose or special purpose computer. Combinations of the above should also be included within the scope of computer-readable media.

Further, upon reaching various computer system components, program code means in the form of computer-executable instructions or data structures can be transferred automatically from transmission media to non-transitory computer-readable storage media (devices) (or vice versa). For example, computer-executable instructions or data structures received over a network or data link can be buffered in RAM within a network interface module (e.g., a NIC), and then eventually transferred to computer system RAM and/or to less volatile computer storage media (devices) at a computer system. Thus, it should be understood that non-transitory computer-readable storage media (devices) can be included in computer system components that also (or even primarily) utilize transmission media.

Computer-executable instructions comprise, for example, instructions and data which, when executed at a processor, cause a general-purpose computer, special purpose computer, or special purpose processing device to perform a certain function or group of functions. In some embodiments, computer-executable instructions are executed on a general-purpose computer to turn the general-purpose computer into a special purpose computer implementing elements of the disclosure. The computer executable instructions may be, for example, binaries, intermediate format instructions such as assembly language, or even source code. Although the subject matter has been described in language specific to structural features and/or methodological acts, it is to be understood that the subject matter defined in the appended claims is not necessarily limited to the described features or acts described above. Rather, the described features and acts are disclosed as example forms of implementing the claims.

Those skilled in the art will appreciate that the disclosure may be practiced in network computing environments with many types of computer system configurations, including, personal computers, desktop computers, laptop computers, message processors, hand-held devices, multiprocessor systems, microprocessor-based or programmable consumer electronics, network PCs, minicomputers, mainframe computers, mobile telephones, PDAs, tablets, pagers, routers, switches, and the like. The disclosure may also be practiced in distributed system environments where local and remote computer systems, which are linked (either by hardwired data links, wireless data links, or by a combination of hardwired and wireless data links) through a network, both perform tasks. In a distributed system environment, program modules may be located in both local and remote memory storage devices.

Embodiments of the present disclosure can also be implemented in cloud computing environments. In this description, "cloud computing" is defined as a model for enabling on-demand network access to a shared pool of configurable computing resources. For example, cloud computing can be employed in the marketplace to offer ubiquitous and convenient on-demand access to the shared pool of configurable computing resources. The shared pool of configurable computing resources can be rapidly provisioned via virtualization and released with low management effort or service provider interaction, and then scaled accordingly.

A cloud-computing model can be composed of various characteristics such as, for example, on-demand self-service, broad network access, resource pooling, rapid elasticity, measured service, and so forth. A cloud-computing model can also expose various service models, such as, for example, Software as a Service (SaaS), Platform as a Service (PaaS), and Infrastructure as a Service (IaaS). A cloud-computing model can also be deployed using different deployment models such as private cloud, community cloud, public cloud, hybrid cloud, and so forth. In this description and in the claims, a "cloud-computing environment" is an environment in which cloud computing is employed.

FIG. 10 illustrates a block diagram of a computing device 1000 that may be configured to perform one or more of the processes described above. One will appreciate that one or more computing devices such as the computing device 1000 may implement the multiomics sequencing system 106 and the multiomics sequencing system 106. As shown by FIG. 10, the computing device 1000 can comprise a processor 1002, a memory 1004, a storage device 1006, an I/O interface 1008, and a communication interface 1010, which may be communicatively coupled by way of a communication infrastructure 1012. In certain embodiments, the computing device 1000 can include fewer or more components than those shown in FIG. 10. The following paragraphs describe components of the computing device 1000 shown in FIG. 10 in additional detail.

In one or more embodiments, the processor 1002 includes hardware for executing instructions, such as those making up a computer program. As an example, and not by way of limitation, to execute instructions for dynamically modifying workflows, the processor 1002 may retrieve (or fetch) the instructions from an internal register, an internal cache, the memory 1004, or the storage device 1006 and decode and execute them. The memory 1004 may be a volatile or non-volatile memory used for storing data, metadata, and programs for execution by the processor(s). The storage device 1006 includes storage, such as a hard disk, flash disk drive, or other digital storage device, for storing data or instructions for performing the methods described herein.

The I/O interface 1008 allows a user to provide input to, receive output from, and otherwise transfer data to and receive data from computing device 1000. The I/O interface 1008 may include a mouse, a keypad or a keyboard, a touch screen, a camera, an optical scanner, network interface, modem, other known I/O devices or a combination of such I/O interfaces. The I/O interface 1008 may include one or more devices for presenting output to a user, including, but not limited to, a graphics engine, a display (e.g., a display screen), one or more output drivers (e.g., display drivers), one or more audio speakers, and one or more audio drivers. In certain embodiments, the I/O interface 1008 is configured to provide graphical data to a display for presentation to a user. The graphical data may be representative of one or more graphical user interfaces and/or any other graphical content as may serve a particular implementation.

The communication interface 1010 can include hardware, software, or both. In any event, the communication interface 1010 can provide one or more interfaces for communication (such as, for example, packet-based communication) between the computing device 1000 and one or more other computing devices or networks. As an example, and not by way of limitation, the communication interface 1010 may include a network interface controller (NIC) or network adapter for communicating with an Ethernet or other wire-based network or a wireless NIC (WNIC) or wireless adapter for communicating with a wireless network, such as a WI-FI.

Additionally, the communication interface 1010 may facilitate communications with various types of wired or wireless networks. The communication interface 1010 may also facilitate communications using various communication protocols. The communication infrastructure 1012 may also include hardware, software, or both that couples components of the computing device 1000 to each other. For example, the communication interface 1010 may use one or more networks and/or protocols to enable a plurality of computing devices connected by a particular infrastructure to communicate with each other to perform one or more aspects of the processes described herein. To illustrate, the sequencing process can allow a plurality of devices (e.g., a client device, sequencing device, and server device(s)) to exchange information such as sequencing data and error notifications.

In the foregoing specification, the present disclosure has been described with reference to specific exemplary embodiments thereof. Various embodiments and aspects of the present disclosure(s) are described with reference to details discussed herein, and the accompanying drawings illustrate the various embodiments. The description above and drawings are illustrative of the disclosure and are not to be construed as limiting the disclosure. Numerous specific details are described to provide a thorough understanding of various embodiments of the present disclosure.

The present disclosure may be embodied in other specific forms without departing from its essential characteristics. The described embodiments are to be considered in all respects only as illustrative and not restrictive. For example, the methods described herein may be performed with less or more steps/acts or the steps/acts may be performed in differing orders. Additionally, the steps/acts described herein may be repeated or performed in parallel with one another or in parallel with different instances of the same or similar steps/acts. The scope of the present application is, therefore, indicated by the appended claims rather than by the foregoing description. All changes that come within the meaning and range of equivalency of the claims are to be embraced within their scope.

## Claims

1. A computer-implemented method comprising:
identifying, for a sample and utilizing a multiomics executable file comprising instructions in a programming language that a computing device can directly execute, transcriptomic reads comprising a first set of cellular barcode sequences representing candidate cells and genomic reads comprising a second set of cellular barcode sequences representing candidate cells;
aligning, utilizing the multiomics executable file, the transcriptomic reads with a reference genome and the genomic reads with the reference genome;
determining counts of aligned transcriptomic reads and counts of aligned genomic reads for target nucleotide sequences within the candidate cells;
selecting, utilizing the multiomics executable file, a subset of candidate cells corresponding to a subset of cellular barcode sequences based on the counts of aligned transcriptomic reads and the counts of aligned genomic reads for target nucleotide sequences within the candidate cells; and
generating, for the sample and utilizing the multiomics executable file, single-cell multiomics outputs for individual cells of the selected subset of candidate cells based on the counts of aligned transcriptomic reads and the counts of aligned genomic reads.

2. The computer-implemented method of claim 1, wherein selecting the subset of candidate cells corresponding to the subset of cellular barcode sequences comprises:
determining, for each target nucleotide sequence within each candidate cell, a first count of aligned transcriptomic reads and a second count of aligned genomic reads; and
clustering, from the first set of cellular barcode sequences and the second set of cellular barcode sequences, cellular barcode sequences in a selected cluster of candidate cells and a non-selected cluster of candidate cells based on the first count of aligned transcriptomic reads and the second count of aligned genomic reads for each target nucleotide sequence within each candidate cell.

3. The computer-implemented method of claim 2, wherein:
determining the first count of aligned transcriptomic reads comprises determining, for each gene encoded by a nucleotide sequence within each candidate cell, a count of unique molecular identifier (UMI) sequences corresponding to aligned genomic reads; and
determining the second count of aligned genomic reads comprises determining, for each accessible genomic region corresponding to a read-coverage peak within each candidate cell, a count of read fragments from aligned genomic reads.

4. The computer-implemented method of claim 2, wherein clustering the cellular barcode sequences comprises clustering the cellular barcode sequences into the selected cluster of candidate cells and a non-selected cluster of candidate cells based on a first dimension for summed counts of aligned transcriptomic reads for each candidate cell and a second dimension for summed counts of aligned genomic reads for each candidate cell.

5. The computer-implemented method of claim 2, further comprising selecting, as part of the subset of candidate cells, a candidate cell grouped together with the selected cluster of candidate cells.

6. The computer-implemented method of claim 2, further comprising not selecting, as part of the subset of candidate cells, a candidate cell grouped together with the non-selected cluster of candidate cells.

7. The computer-implemented method of any one of claims 1-6, wherein aligning the transcriptomic reads and the genomic reads with the reference genome comprises:
configuring a configurable processor to execute a first alignment model that aligns the genomic reads with the reference genome; and
configuring the configurable processor to execute a second alignment model that aligns the transcriptomic reads with the reference genome.

8. The computer-implemented method of any one of claims 1-7, wherein selecting the subset of candidate cells corresponding to the subset of cellular barcode sequences comprises:
storing, on random-access memory, the counts of aligned transcriptomic reads and the counts of aligned genomic reads for the target nucleotide sequences; and
selecting the subset of candidate cells corresponding to the subset of cellular barcode sequences based on the counts of aligned transcriptomic reads and the counts of aligned genomic reads stored on the random-access memory.

9. The computer-implemented method of any one of claims 1-8, wherein the first set of cellular barcode sequences differs from the second set of cellular barcode sequences, and the first set of cellular barcode sequences and the second set of cellular barcode sequences correspond to a same set of candidate cells.

10. The computer-implemented method of any one of claims 1-9, wherein:
the transcriptomic reads comprise a sequence of complementary DNA synthesized from single-stranded ribonucleic acid (RNA) from the sample; and
the genomic reads comprise a nucleotide sequence of genomic deoxyribonucleic acid (DNA) complementing a genomic sequence from the sample.

11. The computer-implemented method of any one of claims 1-10, wherein the genomic reads comprise Assay for Transposase-Accessible Chromatin (ATAC) reads for the sample.

12. The computer-implemented method of any one of claims 1-11, wherein generating the single-cell multiomics outputs for individual cells comprises generating a joint cell-by-feature matrix comprising both single-cell counts of aligned transcriptomic reads and single-cell counts of aligned genomic reads for target nucleotide sequences organized by each candidate cell within the selected subset of candidate cells.

13. The computer-implemented method of any one of claims 1-12, wherein generating the single-cell multiomics outputs for individual cells comprises:
generating a first set of single-cell metrics indicating gene expression for each candidate cell of the selected subset of candidate cells based on the counts of aligned transcriptomic reads; and
generating a second set of single-cell metrics indicating accessible genomic deoxyribonucleic acid (DNA) corresponding to open chromatin for each candidate cell of the selected subset of candidate cells based on the counts of aligned genomic reads.

14. A system comprising at least one processor and a non-transitory computer readable medium comprising instructions that, when executed by the at least one processor, cause the system to perform a method according to any one of claims 1-13.

15. A non-transitory computer-readable medium storing instructions that, when executed by at least one processor, cause a computing device to perform a method according to any one of claims 1-13.

## Patentansprüche

1. Computerimplementiertes Verfahren, umfassend:
Identifizieren, für eine Probe und unter Verwendung einer ausführbaren Multiomics-Datei, die Anweisungen in einer Programmiersprache umfasst, die eine Rechenvorrichtung direkt ausführen kann, transkriptomischer Reads, die einen ersten Satz zellulärer Barcode-Sequenzen umfassen, die Kandidatenzellen repräsentieren, und genomischer Reads, die einen zweiten Satz zellulärer Barcode-Sequenzen umfassen, die Kandidatenzellen repräsentieren;
Ausrichten, unter Verwendung der ausführbaren Multiomics-Datei, der transkriptomischen Reads mit einem Referenzgenom und der genomischen Reads mit dem Referenzgenom;
Bestimmen einer Anzahl ausgerichteter transkriptomischer Reads und einer Anzahl ausgerichteter genomischer Reads für Zielnukleotidsequenzen innerhalb der Kandidatenzellen;
Auswählen, unter Verwendung der ausführbaren Multiomics-Datei, einer Teilmenge von Kandidatenzellen, die einer Teilmenge zellulärer Barcode-Sequenzen entsprechen, basierend auf der Anzahl ausgerichteter transkriptomischer Reads und der Anzahl ausgerichteter genomischer Reads für Zielnukleotidsequenzen innerhalb der Kandidatenzellen; und
Erzeugen, für die Probe und unter Verwendung der ausführbaren Multiomics-Datei, von Einzelzellen-Multiomics-Ausgaben für individuelle Zellen der ausgewählten Teilmenge von Kandidatenzellen basierend auf der Anzahl ausgerichteter transkriptomischer Reads und der Anzahl ausgerichteter genomischer Reads.

2. Computerimplementiertes Verfahren nach Anspruch 1, wobei Auswählen der Teilmenge von Kandidatenzellen, die der Teilmenge von zellulären Barcode-Sequenzen entsprechen, Folgendes umfasst:
Bestimmen, für jede Zielnukleotidsequenz in jeder Kandidatenzelle, einer ersten Anzahl ausgerichteter transkriptomischer Reads und einer zweiten Anzahl ausgerichteter genomischer Reads; und
Clustern, ausgehend von der ersten Menge zellulärer Barcode-Sequenzen und der zweiten Menge zellulärer Barcode-Sequenzen, zellulärer Barcode-Sequenzen in einem ausgewählten Cluster von Kandidatenzellen und einem nicht ausgewählten Cluster von Kandidatenzellen basierend auf der ersten Anzahl ausgerichteter transkriptomischer Reads und der zweiten Anzahl ausgerichteter genomischer Reads für jede Zielnukleotidsequenz innerhalb jeder Kandidatenzelle.

3. Computerimplementiertes Verfahren nach Anspruch 2, wobei:
Bestimmen der ersten Anzahl ausgerichteter transkriptomischer Reads Bestimmen, für jedes von einer Nukleotidsequenz in jeder Kandidatenzelle codierte Gen, einer Anzahl eindeutiger molekularer Identifikator- (UMI) Sequenzen entsprechend ausgerichteten genomischen Reads umfasst; und
Bestimmen der zweiten Anzahl ausgerichteter genomischer Reads Bestimmen, für jede zugängliche genomische Region entsprechend einer Read-Abdeckungsspitze in jeder Kandidatenzelle, einer Anzahl von Read-Fragmenten aus ausgerichteten genomischen Reads umfasst.

4. Computerimplementiertes Verfahren nach Anspruch 2, wobei Clustern der zellulären Barcode-Sequenzen Clustern der zellulären Barcode-Sequenzen in den ausgewählten Cluster von Kandidatenzellen und einen nicht ausgewählten Cluster von Kandidatenzellen umfasst, basierend auf einer ersten Dimension für summierte Anzahlen ausgerichteter transkriptomischer Reads für jede Kandidatenzelle und einer zweiten Dimension für summierte Anzahlen ausgerichteter genomischer Reads für jede Kandidatenzelle.

5. Computerimplementiertes Verfahren nach Anspruch 2, ferner umfassend Auswählen, als Teil der Teilmenge von Kandidatenzellen, einer Kandidatenzelle, die mit dem ausgewählten Cluster von Kandidatenzellen gruppiert ist.

6. Computerimplementiertes Verfahren nach Anspruch 2, ferner umfassend Nicht-Auswählen, als Teil der Teilmenge von Kandidatenzellen, einer Kandidatenzelle, die mit dem nicht ausgewählten Cluster von Kandidatenzellen gruppiert ist.

7. Computerimplementiertes Verfahren nach einem der Ansprüche 1-6, wobei Ausrichten der transkriptomischen Reads und der genomischen Reads mit dem Referenzgenom Folgendes umfasst:
Konfigurieren eines konfigurierbaren Prozessors, ein erstes Ausrichtungsmodell auszuführen, das die genomischen Reads mit dem Referenzgenom ausrichtet; und
Konfigurieren des konfigurierbaren Prozessors, ein zweites Ausrichtungsmodell auszuführen, das die transkriptomischen Reads mit dem Referenzgenom ausrichtet.

8. Computerimplementiertes Verfahren nach einem der Ansprüche 1-7, wobei Auswählen der Teilmenge von Kandidatenzellen, die der Teilmenge von zellulären Barcode-Sequenzen entsprechen, Folgendes umfasst:
Speichern, auf einem Direktzugriffspeicher, der Anzahl ausgerichteter transkriptomischer Reads und der Anzahl ausgerichteter genomischer Reads für die Zielnukleotidsequenzen; und
Auswählen der Teilmenge von Kandidatenzellen, die der Teilmenge zellulärer Barcode-Sequenzen entsprechen, basierend auf der Anzahl ausgerichteter transkriptomischer Reads und der Anzahl ausgerichteter genomischer Reads, die in dem Direktzugriffsspeicher gespeichert sind.

9. Computerimplementiertes Verfahren nach einem der Ansprüche 1-8, wobei sich die erste Menge zellulärer Barcode-Sequenzen von der zweiten Menge zellulärer Barcode-Sequenzen unterscheidet und die erste Menge zellulärer Barcode-Sequenzen und die zweite Menge zellulärer Barcode-Sequenzen einem selben Satz von Kandidatenzellen entsprechen.

10. Computerimplementiertes Verfahren nach einem der Ansprüche 1-9, wobei:
die transkriptomischen Daten eine Sequenz komplementärer DNA umfassen, die aus einzelsträngiger Ribonukleinsäure (RNA) der Probe synthetisiert wurde; und
die genomischen Reads eine Nukleotidsequenz genomischer Desoxyribonukleinsäure (DNA) umfassen, die eine genomische Sequenz aus der Probe komplementiert.

11. Computerimplementiertes Verfahren nach einem der Ansprüche 1-10, wobei die genomischen Reads Assay for Transposase-Accessible Chromatin (ATAC) Reads für die Probe umfassen.

12. Computerimplementiertes Verfahren nach einem der Ansprüche 1-11, wobei Erzeugen der Einzelzellen-Multiomics-Ausgaben für individuelle Zellen Erzeugen einer gemeinsamen Zelle-nach-Merkmal-Matrix umfasst, die sowohl Einzelzellzählungen ausgerichteter transkriptomischer Reads als auch Einzelzellzählungen ausgerichteter genomischer Reads für Zielnukleotidsequenzen umfasst, die nach jeder Kandidatenzelle innerhalb der ausgewählten Teilmenge von Kandidatenzellen organisiert sind.

13. Computerimplementiertes Verfahren nach einem der Ansprüche 1-12, wobei Erzeugen der Einzelzell-Multiomics-Ausgaben für individuelle Zellen Folgendes umfasst:
Erzeugen einer ersten Menge von Einzelzellmetriken, die eine Genexpression für jede Kandidatenzelle der ausgewählten Teilmenge von Kandidatenzellen basierend auf der Anzahl ausgerichteter transkriptomischer Reads angeben; und
Erzeugen einer zweiten Menge von Einzelzellmetriken, die die zugängliche genomische Desoxyribonukleinsäure (DNA) entsprechend offenem Chromatin für jede Kandidatenzelle der ausgewählten Teilmenge von Kandidatenzellen basierend auf der Anzahl ausgerichteter genomischer Reads angibt.

14. System, das mindestens einen Prozessor und ein nichtflüchtiges, computerlesbares Medium umfasst, das Anweisungen umfasst, die, wenn sie von dem mindestens einen Prozessor ausgeführt werden, das System veranlassen, ein Verfahren nach einem der Ansprüche 1-13 durchzuführen.

15. Nichtflüchtiges, computerlesbares Medium, das Anweisungen speichert, die, wenn sie von mindestens einem Prozessor ausgeführt werden, eine Rechenvorrichtung veranlassen, ein Verfahren nach einem der Ansprüche 1-13 durchzuführen.

## Revendications

1. Procédé mis en œuvre par ordinateur comprenant :
l'identification, pour un échantillon et en utilisant un fichier exécutable multiomique comprenant des instructions dans un langage de programmation qu'un dispositif informatique peut exécuter directement, de lectures transcriptomiques comprenant un premier ensemble de séquences de codes-barres cellulaires représentant des cellules candidates et des lectures génomiques comprenant un second ensemble de séquences de codes-barres cellulaires représentant des cellules candidates ;
l'alignement, en utilisant le fichier exécutable multiomique, des lectures transcriptomiques avec un génome de référence et des lectures génomiques avec le génome de référence ;
la détermination de comptes de lectures transcriptomiques alignées et de comptes de lectures génomiques alignées pour des séquences nucléotidiques au sein des cellules candidates ;
la sélection, en utilisant le fichier exécutable multiomique, d'un sous-ensemble de cellules candidates correspondant à un sous-ensemble de séquences de codes-barres cellulaires sur la base des comptes de lectures transcriptomiques alignées et des comptes de lectures génomiques alignées pour des séquences nucléotidiques cibles au sein des cellules candidates ; et
la génération, pour l'échantillon et en utilisant le fichier exécutable multiomique, de sorties multiomiques unicellulaires pour des cellules individuelles du sous-ensemble sélectionné de cellules candidates sur la base des comptes de lectures transcriptomiques alignées et des comptes de lectures génomiques alignées.

2. Procédé mis en œuvre par ordinateur selon la revendication 1, dans lequel la sélection du sous-ensemble de cellules candidates correspondant au sous-ensemble de séquences de codes-barres cellulaires comprend :
la détermination, pour chaque séquence nucléotidique cible au sein de chaque cellule candidate, d'un premier compte de lectures transcriptomiques alignées et d'un second compte de lectures génomiques alignées ; et
le regroupement, à partir du premier ensemble de séquences de codes-barres cellulaires et du second ensemble de séquences de codes-barres cellulaires, de séquences de codes-barres cellulaires dans un groupe sélectionné de cellules candidates et d'un groupe non sélectionné de cellules candidates sur la base du premier compte de lectures transcriptomiques alignées et du second compte de lectures génomiques alignées pour chaque séquence nucléotidique cible au sein de chaque cellule candidate.

3. Procédé mis en œuvre par ordinateur selon la revendication 2, dans lequel :
la détermination du premier compte de lectures transcriptomiques alignées comprend la détermination, pour chaque gène codé par une séquence nucléotidique au sein de chaque cellule candidate, d'un compte de séquences d'identifiant moléculaire unique (UMI) correspondant à des lectures génomiques alignées ; et
la détermination du second compte de lectures génomiques alignées comprend la détermination, pour chaque région génomique accessible correspondant à un pic de couverture de lecture au sein de chaque cellule candidate, d'un compte de fragments de lecture à partir de lectures génomiques alignées.

4. Procédé mis en œuvre par ordinateur selon la revendication 2, dans lequel le regroupement des séquences de code-barres cellulaires comprend le regroupement des séquences de code-barres cellulaires en le groupe sélectionné de cellules candidates et un groupe non sélectionné de cellules candidates sur la base d'une première dimension pour des comptes cumulés de lectures transcriptomiques alignées pour chaque cellule candidate et d'une seconde dimension pour des comptes cumulés de lectures génomiques alignées pour chaque cellule candidate.

5. Procédé mis en œuvre par ordinateur selon la revendication 2, comprenant en outre la sélection, dans le cadre du sous-ensemble de cellules candidates, d'une cellule candidate regroupée avec le groupe sélectionné de cellules candidates.

6. Procédé mis en œuvre par ordinateur selon la revendication 2, comprenant en outre la non-sélection, dans le cadre du sous-ensemble de cellules candidates, d'une cellule candidate regroupée avec le groupe de cellules candidates non sélectionné.

7. Procédé mis en œuvre par ordinateur selon l'une quelconque des revendications 1-6, dans lequel l'alignement des lectures transcriptomiques et des lectures génomiques avec le génome de référence comprend :
la configuration d'un processeur configurable pour exécuter un premier modèle d'alignement qui aligne les lectures génomiques avec le génome de référence ; et
la configuration du processeur configurable pour exécuter un second modèle d'alignement qui aligne les lectures transcriptomiques avec le génome de référence.

8. Procédé mis en œuvre par ordinateur selon l'une quelconque des revendications 1-7, dans lequel la sélection du sous-ensemble de cellules candidates correspondant au sous-ensemble de séquences de codes-barres cellulaires comprend :
le stockage, en mémoire à accès aléatoire, du compte de lectures transcriptomiques alignées et du compte de lectures génomiques alignées pour les séquences nucléotidiques cibles ; et
la sélection du sous-ensemble de cellules candidates correspondant au sous-ensemble de séquences de codes-barres cellulaires sur la base du compte de lectures transcriptomiques alignées et des comptes de lectures génomiques alignées stockées dans la mémoire à accès aléatoire.

9. Procédé mis en œuvre par ordinateur selon l'une quelconque des revendications 1-8, dans lequel le premier ensemble de séquences de codes-barres cellulaires diffère du second ensemble de séquences de codes-barres cellulaires, et le premier ensemble de séquences de codes-barres cellulaires et le second ensemble de séquences de codes-barres cellulaires correspondent à un même ensemble de cellules candidates.

10. Procédé mis en œuvre par ordinateur selon l'une quelconque des revendications 1-9, dans lequel :
les lectures transcriptomiques comprennent une séquence d'ADN complémentaire synthétisée à partir d'acide ribonucléique (ARN) simple brin à partir de l'échantillon ; et
les lectures génomiques comprennent une séquence nucléotidique d'acide désoxyribonucléique (ADN) génomique complémentaire d'une séquence génomique à partir de l'échantillon.

11. Procédé mis en œuvre par ordinateur selon l'une quelconque des revendications 1-10, dans lequel les lectures génomiques comprennent des lectures Assay for Transposase-Accessible Chromatin (ATAC) pour l'échantillon.

12. Procédé mis en œuvre par ordinateur selon l'une quelconque des revendications 1-11, dans lequel la génération des sorties multiomiques unicellulaires pour des cellules individuelles comprend la génération d'une matrice cellule par caractéristique conjointe comprenant à la fois des comptes unicellulaires de lectures transcriptomiques alignées et des comptes unicellulaires de lectures génomiques alignées pour des séquences nucléotidiques cibles organisées par chaque cellule candidate au sein du sous-ensemble sélectionné de cellules candidates.

13. Procédé mis en œuvre par ordinateur selon l'une quelconque des revendications 1-12, dans lequel la génération des sorties multiomiques unicellulaires pour des cellules individuelles comprend :
la génération d'un premier ensemble de mesures unicellulaires indiquant l'expression génique pour chaque cellule candidate du sous-ensemble sélectionné de cellules candidates sur la base des comptes de lectures transcriptomiques alignées ; et
la génération d'un second ensemble de mesures unicellulaires indiquant de l'acide désoxyribonucléique (ADN) génomique accessible correspondant à de la chromatine ouverte pour chaque cellule candidate du sous-ensemble sélectionné de cellules candidates sur la base des comptes de lectures génomiques alignées.

14. Système comprenant au moins un processeur et un support lisible par ordinateur non transitoire comprenant des instructions qui, lorsqu'elles sont exécutées par l'au moins un processeur, amènent le système à réaliser un procédé selon l'une quelconque des revendications 1-13.

15. Support lisible par ordinateur non transitoire stockant des instructions qui, lorsqu'elles sont exécutées par au moins un processeur, amènent un dispositif informatique à réaliser un procédé selon l'une quelconque des revendications 1-13.
